(19) Europäisches Patentamt
European Patent Office
Office européen des brevets

(11) **EP 4 201 507 A1**

(12) **EUROPEAN PATENT APPLICATION**

(43) Date of publication:
**28.06.2023 Bulletin 2023/26**

(21) Application number: **21216677.1**

(22) Date of filing: **21.12.2021**

(51) International Patent Classification (IPC):
***B01D 67/00*** *(2006.01)* ***A61M 1/34*** *(2006.01)*
***B01D 71/44*** *(2006.01)* ***B01D 71/68*** *(2006.01)*

(52) Cooperative Patent Classification (CPC):
**B01D 71/68; A61M 1/1621; B01D 67/0088; B01D 67/0093; B01D 71/441;** B01D 2313/68; B01D 2323/2187

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Gambro Lundia AB**
  **220 10 Lund (SE)**
- **RWTH Aachen**
  **52062 Aachen (DE)**

(72) Inventors:
- **SWARTJES, Jan J.T.M.**
  **72072 Tübingen (DE)**
- **STORR, Markus**
  **70794 Filderstadt (DE)**
- **VOTTELER, Stefanie**
  **72768 Reutlingen (DE)**
- **HORNUNG, Markus**
  **72147 Nehren (DE)**
- **HULKO, Michael**
  **71149 Bondorf (DE)**
- **KRAUß, Annabelle**
  **72661 Grafenberg (DE)**
- **PETERS, Lars**
  **4852 Weyregg am Attersee (AT)**
- **WESSLING, Matthias**
  **52074 Aachen (DE)**
- **ROSE, Ilka**
  **52074 Aachen (DE)**
- **LINZ, Georg**
  **52064 Aachen (DE)**
- **ROTH, Hannah**
  **52062 Aachen (DE)**

(74) Representative: **Hornung, Veronika Margot**
**Gambro Dialysatoren GmbH**
**Holger-Crafoord-Strasse 26**
**72379 Hechingen (DE)**

# (54) METHOD FOR INCREASING THE SELECTIVITY OF A MEMBRANE

(57) The disclosure relates to a method for improving the selectivity of a membrane, specifically of a membrane comprising a blend of i) a polysulfone, polyethersulfone or polyarylethersulfone and ii) polyvinylpyrrolidone, characterized in that the membrane is coated with polydopamine and heparin in a one-step or two-step reaction or by in situ modification with polydopamine followed by coating with heparin. The disclosure further relates to a process for producing such modified membranes having an improved selectivity, and to a filtration/diffusion device comprising the membrane which can be used, for example, in hemodialysis applications.

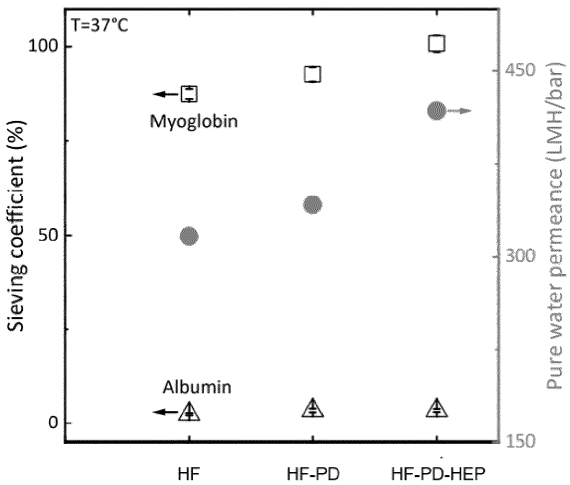


Figure 5



Processed by Luminess, 75001 PARIS (FR)

## Description

### Technical Field

The disclosure relates to a method for improving the selectivity of a membrane, specifically of a membrane comprising a blend of i) a polysulfone, polyethersulfone or polyarylethersulfone and ii) polyvinylpyrrolidone, characterized in that the membrane is coated with polydopamine and heparin in a one-step or two-step reaction or by in situ modification with polydopamine followed by coating with heparin. The disclosure further relates to a process for producing such modified membranes having an improved selectivity, and to a filtration/diffusion device comprising the membrane which can be used, for example, in hemodialysis applications.

### Description of the Related Art

Patients with end stage renal disease in hemodialysis have an increased mortality risk when compared to the general population. This indicates that the currently available replacement therapies, even though relying on advanced membrane and dialyzer technologies as well as highly effective and sophisticated machines, should be further improved in a way that the clinical outcome for patients and their quality of life during therapy is getting better. One of the still manifold unmet needs that has been accompanying the development of new membranes and dialyzers over decades is the enhanced removal of uremic toxins over a broad range, while essentially retaining proteins such as albumin and higher molecular weight compounds that are deemed to be critical. This matter, despite being a target of research since many years and despite significant improvements made, remains a persistent problem because synthetic membranes available today are still less selective than the glomerular membrane of the kidney, and so higher molecular weight uremic toxins of up to the size of about 58 kDa are not cleared from the patients' blood appropriately where larger molecules having a higher molecular weight, such as albumin, must be retained at the same time. Also, hemodialysis is performed generally thrice weekly for approximately 4 hours, while the healthy kidney operates continuously (Boschetti-de-Fierro et al., Scientific Reports (2015) 5: 18448), which adds to the issues around the accumulation of uremic toxins in the body. Another persistent need in hemodialysis is the hemocompatibility of the materials used in the treatment, specifically the hemocompatibility of membranes that are in direct blood contact, as well as their thrombogenicity that requires using systemic heparin in most therapies.

Accordingly, membrane innovation has recently been focused on the enhanced removal of larger molecular weight uremic toxins and increased membrane permeability, and the improvement of hemocompatibility and antithrombogenicity of the membranes. The progressive shift towards increased membrane permeability and concomitant increased selectivity is driven by the persistent gap between synthetic membranes and the natural kidney which combines both high permeability and high selectivity.

As mentioned above, typical hallmarks of selectivity of a membrane specifically for use in hemodialysis are the sieving coefficients for albumin (~66.4kDa) on the one hand and the sieving coefficients for large middle molecules (>25-58kDa) on the other hand (Rosner et al., Classification of Uremic Toxins and Their Role in Kidney Failure: CJASN 16, 1-8 (2021). Such large middle molecules include, for example, YKL-40 (~40kDa), AGEs, lambda-FLC, CX3CL1, CXCL12, or IL-2. However, selectivity does expressly encompass the highly efficient removal of also small middle molecules (0.5-15kDa) such as β2-microglubulin, and medium middle molecules (>15-25kDa), such as TNF, IL-10, IL-6, kappa-FLC, myoglobin, FGF-2, complement factor D and others. The sieving coefficients for said molecules are also reflected in the sieving curve as represented by the MWCO and MWRO values of a membrane which provide information on the onset of retention of molecules, i.e., where the sieving coefficient is 0.9, and the cut-off of a membrane, i.e., where the sieving coefficient is 0.1, and which, therefore, provide for an excellent measure of the retention onset and cut-off of a membrane and, consequently, its selectivity. For example, shifting the retention onset to higher molecular weights while keeping the cut-off stable or even reducing it results in increased selectivity, which is apparent from an increased steepness of the sieving curve **(Figure 1)**. Alternatively, keeping the MWRO of a given membrane stable while reducing its MWCO is another good way to increase the selectivity of the membrane.

Hemodialyzers comprising membranes with an improved selectivity which can, for example, be described with the beforementioned MWRO and MWCO values, should have a low albumin loss over a typical dialysis treatment of about 4 hours (or simulated use correlated with *in vivo* use), i.e., the albumin loss should remain below about 7 g/4h, and preferably should remain below about 4g/4h. At the same time, such hemodialyzers should provide for a good clearance for said larger middle molecules, including, for example, YKL-40, i.e., they should have a YKL-40 clearance of at least 20 mL/min and higher. They should efficiently remove β-2 microglobulin (β2m) with a clearance of more than 80 mL/min.

β-2 microglobulin is a well-known marker molecule in hemodialysis. It is a component of MHC class I molecules with a molecular weight of 11kDa that in patients on long-term hemodialysis can aggregate into amyloid fibers that deposit in joint spaces, a disease, known as dialysis-related amyloidosis. YKL-40 (or chitinase-3-like protein) has recently

emerged as a relevant and functional marker protein in hemodialysis (Lorenz et al., Kidney International (2018) 93: 221-230), where it has been linked to chronic inflammatory response in chronic kidney disease and HD patients, and where its presence in the serum of the patients makes it accessible and traceable.

Recently, a significant step towards membranes and hemodialyzers with an improved performance was made by the introduction of so-called MCO membranes and dialyzers, which are characterized by membranes having a high molecular weight retention onset (MWRO), thereby allowing also larger molecular weight toxins to pass the membrane to a significant extent, combined with a sufficiently low molecular weight cut off (MWCO) to make sure that desired proteins that have a molecular weight of albumin and higher are essentially retained (WO 2015/118046A1 and WO 2015/118046A1). In other words, the design of the membrane allows for a significantly improved selectivity, which is a distinguishing feature over other prior art membranes such as the so-called "high cut-off" (HCO) membranes or membranes that have been referred to, in the past, as "protein-leaking" (PL) membranes. While HCO membranes are also able to efficiently remove the said higher molecular weight toxins, their increased albumin loss recommends their use only in acute situations that are limited in time and strictly controlled as regards albumin loss and replacement thereof. Protein-leaking membranes, on the other hand, generally have a cut-off that reasonably limits albumin loss, but the retention onset is relatively low, resulting in flat sieving curves that signify a low selectivity. In contrast, the very selective MCO membranes when combined with a careful physical design of the hollow fibers, membrane bundles and dialyzers lead to a performance which provides for an unprecedented clearance of relevant higher molecular weight toxins in hemodialysis, while albumin loss can be kept within narrow limits (Kirsch et al., Nephrology Dialysis Transplantation (2017) 32: 165.

The significant contribution of said MCO membranes and dialyzer design to a superior dialyzer performance was achieved by a careful control of all relevant parameters of the manufacturing process as described in WO 2015/118046A1 and WO 2015/118046A1. Such highly controlled manufacturing process is not easily reproducible throughout the industry and further improving the selectivity of the said MCO membranes by means of the process as such is challenging and/or costly. Accordingly, it would be desirable to further improve membranes towards even higher selectivity by simpler, easily accessible methods that allow the production of highly selective hemodialysis membranes also in simpler settings and without excessive costs that add to the price of the final product.

Other membranes, such as standard hemodialysis membranes and dialyzers, including, for example, high-flux membranes that are today widely used in hemodialysis (HD) could also benefit from methods to improve their selectivity in a simple, cost-effective and safe way to provide access to improved dialyzers to more patients that may otherwise not be able to benefit from the potentially more expensive high-end dialyzers as discussed above or potentially more complex and access-limited therapies such as HDF. Clearly, any such methods for improving the selectivity of available membranes and dialyzers must guarantee the biocompatibility of the membrane and the dialyzer and its general safety for the patient.

The modification of membranes, including hemodialysis membranes, by immobilizing biologically active or inactive compounds, e.g., for improving their biocompatibility, antithrombogenicity, or performance, or for adding certain specific or unspecific properties, e.g., for capturing certain compounds such as, for example, hydrophilic or hydrophobic molecules, or for specifically immobilizing certain molecules by immobilized antibodies against such molecules have been described in the prior art.

For example, US 2003/0021826 A1 proposes binding an anticoagulation agent in a stable manner to the surface of semipermeable support membranes essentially comprised of a copolymer of acrylonitrile and at least one anionic or anionizable monomer. The anticoagulation agent can exert its anticoagulating activity without being leached out into the blood or plasma during treatment by extracorporeal circulation. The surface of the semipermeable support membrane intended to be brought into contact with the blood or plasma is coated in succession with a cationic polymer carrying cationic groups which can form an ionic bond with anionic or anionizable groups of polyacrylonitrile, for example, polyethyleneimine (PEI), and an anticoagulation agent carrying anionic groups capable of forming an ionic bond with cationic groups of said cationic polymer (for example, heparin). Membranes based on this technology are today used in hemodialysis, for example the Evodial or Oxiris dialyzer (both from Baxter) .

Typically, the hemodialysis treatment requires the dosage of heparin to the patient to prevent blood clotting on the artificial membrane surface. Heparin activates antithrombin, which inhibits factor X as well as thrombin. Due to this influence on the coagulation cascade, the formation of a thrombus in the human blood is disturbed. Furthermore, heparin significantly reduces platelet deposition. In clinical treatments, the dosage of heparin is not generally individualized. Therefore, it is administered in larger amounts and results in long waiting times until the heparin is degraded in the patients' blood after each treatment procedure. The immobilization of heparin on the membrane surface is a promising approach to prevent the heparin administration and resulting long-term damages.

Another compound which has been widely used already for modifying membranes alone or in combination with other materials is polydopamine. It has been used for the modification in various membrane processes and for various purposes.

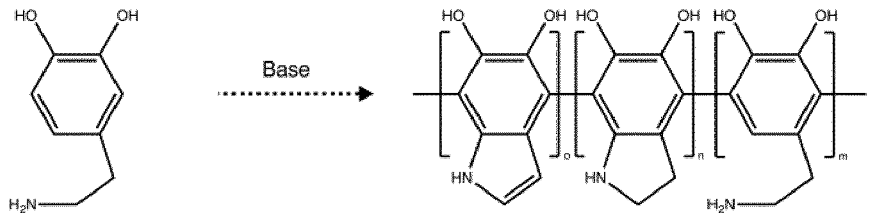


Dopamine Polydopamine

Polydopamine is obtained from dopamine, for example, in an alkaline environment or through oxidation and adheres to various surfaces. The variety of reactive groups provides for many possibilities for functionalizing surfaces for practical applications in material sciences, biomedicine, surface engineering and catalysis (Liebscher, Chemistry of Polydopamine - Scope, Variation, and Limitation. Eur. J. Org. Chem., 2019 (2019): 4976-4994).

For example, WO 2012/047282 A2 describes methods of enhancing the anti-fouling properties of TFC-FO membranes by coating them with a mixture of dopamine and anti-fouling polymers. The addition of dopamine to the reaction mixture enables the formation of a more stable coating. US 2010/0059433 A1 also discloses depositing a coating material on a membrane used for purifying contaminated water to reduce or prevent biofilm formation on the membrane surface by adding a dopamine coating material to a liquid solvent to form a solution mixture, adjusting a pH of the solution mixture to 8, 9, or 10 and dissolving the dopamine coating material in the liquid solvent. The solution mixture is then placed into contact with the membrane surfaces to form a dopamine coating on the surface to reduce biofilm formation.

Li et al., Desalination 344 (2014): 422-430 describe the modification of polyethersulfone (PES) membranes with different MWCO (molecular weight cut offs) by polydopamine (PD) coating and PD-graft-poly(ethylene glycol) (PD-g-PEG) modification, resulting in less flux reduction in filtration and lower adsorptive amount of BSA in isothermal adsorption tests.

Zhu et al., (Surface modification of PVDF porous membranes via poly(DOPA) coating and heparin immobilization, Colloids and Surfaces B: Biointerfaces 69 (1) (2009) 152-155) first combined heparin with polydopamine on poly(vinylidene fluoride) (PVDF) microporous membranes. It was followed by further research in the field of heparin onto polydopamine treated membranes. While Jiang et al. (Surface modification of PE porous membranes based on the strong adhesion of polydopamine and covalent immobilization of heparin, Journal of Membrane Science 364 (1-2) (2010) 194-202) coat polyethylene (PE) porous membranes and show a covalent bond that is created during the coating process, You et al. (Enhancement of blood compatibility of poly (urethane) substrates by mussel-inspired adhesive heparin coating, Bioconjugate Chemistry 22 (7) (2011) 1264-1269) show a significantly inhibited blood clotting by the use of treated poly(urethane) (PU) substrates.

Research into heparinized polydopamine treated membranes has also been carried out in the field of dialysis. Gao et al., Journal of Membrane Science 452 (2014): 390-399 describe the production of a bio-based poly (lactic acid) (PLA) membrane with asymmetric porous structure for hemodialysis to which heparin was immobilized surface via dopamine. The surface heparinization mediated by dopamine improved the hemocompatibility of the PLA membrane, suppressed the adhesion of platelets and reduced hemolysis. Ma et al. (Mussel-inspired self-coating at macro-interface with improved biocompatibility and bioactivity via dopamine grafted heparin-like polymers and heparin, Journal of Materials Chemistry B 2 (2014) 363-375) use PES membranes and show decreased plasma protein adsorption and suppressed platelet adhesion compared to the untreated membrane.

However, it has not been described so far that it is possible to influence the selectivity of a given membrane by applying a tailored coating based on polydopamine and heparin to said base membrane. Specifically, it was only now found that a carefully designed method of coating membranes for blood treatment, including, but not limited to, hemodialysis, hemofiltration or hemodiafiltration, specifically polysulfone, polyethersulfone and polyarylethersulfon based membranes with polydopamine and heparin results in membranes that have an increased selectivity compared to the base membrane without such coating. It was further found that such coating can be applied either as a combination layer of polydopamine and heparin or as separate, consecutive layers of polydopamine followed by heparin. Another option is to add polydopamine to the spinning solution ("*in situ* polydopamine functionalization") of a given base membrane recipe with subsequent immobilization of heparin on the polydopamine modified membrane. The resulting polydopamine-heparin modified membranes (PD-HEP) were found to be stable during a typical hemodialysis treatment and to improve the performance and selectivity of the base membrane, i.e., the membrane without PD-HEP coating or the membrane not comprising polydopamine with additional heparin coating, respectively.

## Summary

It is an object of the present invention to provide a method for improving the selectivity of a hollow fiber and flat sheet membrane for use in blood treatment applications, specifically in hemodialysis, hemofiltration or hemodiafiltration by modifying them with polydopamine and heparin either through coating with a mixture of polydopamine and heparin or subsequent coating with first dopamine followed by heparin, or by *in situ* polydopamine functionalization of the membrane followed by coating with heparin. As used herein, the expression "membrane" or "membranes" encompasses both hollow fiber membranes and flat sheet membranes. In the context of the present invention, hollow fiber membranes are preferred as they constitute the state-of-the-art membrane configuration for use in hemodialysis devices.

Membranes that can, for example, be used as base membranes comprise a blend of i) a polysulfone, a polyethersulfone or a polyarylethersulfone and ii) polyvinylpyrrolidone. Said membranes can have different physical structures, including varying degrees of asymmetric configuration, ranging from minimum asymmetry in sponge-like structures to maximum asymmetry in finger-like structures, such as described, for example, in Ronco and Clark, Nature Reviews. Nephrology 14 (6) (2018): 394-410). In case of hollow fiber membranes, the lumen or inner side of the hollow fibers membranes is preferably coated as it generally provides for the selective layer of the hollow fiber membranes that is in contact with blood, or, optionally, with blood plasma. In devices where the outer side of a hollow fiber membrane is in contact with blood and provides for the selective layer of the membrane, a modification or coating according to the invention would preferably be applied to the outer side of the hollow fiber membranes.

According to one aspect, base membranes for use in a method according to the invention have a MWRO of from 8.5 to 14.0 kDa and a MWCO of from 50.0 to 130.0 kDa as measured by dextran sieving before blood contact, preferably a MWRO of from 8.5 to 12.5 kDa and a MWCO of from 55.0 to 110.0 kDa as measured by dextran sieving before blood contact. According to yet another aspect, the base membranes have a MWRO of from 9.0 to 12.5 kDa and a MWCO of from 55.0 to 90.0 kDa as measured by dextran sieving before blood contact. Such membranes are generally referred to as MCO (medium cut-off) membranes, sometimes also as HRO (high retention onset) membranes. They are described in detail in WO 2015/118045 A1 and WO 2015/118046 A1, respectively. A product which is a representative of such MCO type membrane that is available in the market today is the Theranova dialyzer (Baxter) .

According to another aspect, the base membranes for use in a method according to the invention have a MWRO of from 15 kDa to 20 kDa and a MWCO of from 170 kDa to 320 kDa as measured by dextran sieving before blood contact. Such membranes are described, for example, in WO 2004/056460 A1 or in Gondouin and Hutchison: High Cut-Off Dialysis Membranes: Current Uses and Future Potential. Advances in Chronic Kidney Disease 18 (2011):180-187. A product which is one representative of such HCO type membranes and dialyzers, and which is available in the market today, is the Theralite dialyzer (Baxter).

According to another aspect, the base membrane for use in a method according to the invention is a high-flux (HF) membrane with a MWRO of from 5.0 kDa to 8.5 kDa and a MWCO of from 25 kDa to 50 kDa as measured by dextran sieving before blood contact.

According to one aspect of the invention, the membranes can be coated in one step with a solution comprising dopamine and heparin **(Figure 2B**). For the avoidance of doubt, dopamine is the starting material which is provided in the solution for coating the base membrane and wherein it reacts to polydopamine. Accordingly, as used herein, dopamine refers to the starting material added to the coating solution. It is implied, however, that it is transferred into polydopamine in the process of coating and is present as polydopamine on the final membrane.

According to one aspect, the concentration of dopamine in the coating solution is between 2 mg/mL to 80 mg/mL. The pH of the solution is preferably between 8.0 and 10, such as between 8.0 and 9.8, between 8.0 and 9.6, or between 8.2 and 9.4. According to yet another aspect of the invention the concentration of heparin in the coating solution is between 10 mg/mL and 60 mg/mL, preferably between 15 mg/mL and 50 mg/mL, such as, for example, 15 mg/L, 20 mg/L, 25 mg/L, 30 mg/L, 35 mg/L, 35 mg/L, 40 mg/L, 45 mg/L and 50 mg/L.

According to another aspect, the membranes are coated with polydopamine in an amount of from 0.30 $\mu g/cm^2$ to 1.10 $\mu g/cm^2$ of the membrane surface, such as 0.30 $\mu g/cm^2$, 0.40 $\mu g/cm^2$, 0.50 $\mu g/cm^2$, 0.60 $\mu g/cm^2$, 0.70 $\mu g/cm^2$, 0.80 $\mu g/cm^2$, 0.90 $\mu g/cm^2$, 1.00 $\mu g/cm^2$, 1.10 $\mu g/cm^2$, or 1.20 $\mu g/cm^2$. According to another aspect of the invention, the membranes are coated with heparin in an amount of from 0.50 $\mu g/cm^2$ to 0.70 $\mu g/cm^2$. According to yet another aspect of the invention, the membranes are coated with polydopamine in an amount of from 0.40 $\mu g/cm^2$ to 0.60 $\mu g/cm^2$ of the membrane surface. According to yet another aspect of the invention, the membranes are coated with heparin in an amount of from 0.60 $\mu g/cm^2$ to 1.10 $\mu g/cm^2$.

According to a further aspect, the base membranes **(Figure 2(0)**) are coated with polydopamine and heparin in two consecutive steps, wherein the membranes are first coated with polydopamine **(Figure 2A**) followed by coating with heparin in a second step, thereby providing for coated membranes comprising or consisting of a base membrane which is coated with a first coating layer comprising or essentially consisting of polydopamine, and a second coating layer comprising or essentially consisting of heparin.

According to another aspect, the membranes are modified with polydopamine and heparin by *in situ* modification

of the base membrane with polydopamine in a first step **(Figure 2C**), followed by coating of the polydopamine modified base membrane with heparin in a second step. Polydopamine can also be added according to any of the above processes to flat sheet membranes, i.e., either by an *in situ* casting process or by a one-step or subsequent coating with polydopamine and heparin.

According to another aspect, the concentration of heparin in the coating solution is between 0.1 mg/mL to 100 mg/mL, from 0.5 mg/mL to 80.0 mg/mL, from 1 mg/mL to 60 mg/L, or from 5 mg/mL to 40 mg/L. For example, the concentration of heparin in the coating solution is 0.5 mg/mL, 1 mg/mL, 2 mg/mL, 3 mg/mL, 4 mg/mL, 5 mg/ml, 10 mg/ml, 15 mg/ml, 20 mg/ml, 25 mg/ml, 30 mg/ml, 35 mg/ml, 40 mg/ml, 45 mg/ml, 50 mg/ml, 55 mg/ml, 60 mg/ml, 65 mg/ml, 70 mg/ml, 75 mg/ml, or 80 mg/ml. According to a further aspect, the concentration of heparin in the coating solution is between 10 mg/mL and 50 mg/mL. According to a further aspect, the concentration of heparin in the coating solution is between 0.1 mg/mL to 20 mg/mL, from 0.1 mg/mL to 10 mg/mL or from 0.5 mg/mL to 10 mg/mL.

According to yet another aspect, the membrane is coated with heparin in an amount of from 5 $mU/cm^2$ to 100 $mU/cm^2$ of the membrane surface, from 5 $mU/cm^2$ to 80 $mU/cm^2$ of the membrane surface, from 5 $mU/cm^2$ to 50 $mU/cm^2$ of the membrane surface, or from 5 $mU/cm^2$ to 30 $mU/cm^2$ of the membrane surface, such as, for example 5 $mU/cm^2$, 6 $mU/cm^2$, 7 $mU/cm^2$, 8 $mU/cm^2$, 9 $mU/cm^2$, 10 $mU/cm^2$, 11 $mU/cm^2$, 12 $mU/cm^2$, 13 $mU/cm^2$, 14 $mU/cm^2$, 15 $mU/cm^2$, 16 $mU/cm^2$, 17 $mU/cm^2$, 18 $mU/cm^2$, 19 $mU/cm^2$, 20 $mU/cm^2$, 21 $mU/cm^2$, 22 $mU/cm^2$, 23 $mU/cm^2$, 24 $mU/cm^2$, 25 $mU/cm^2$, 30 $mU/cm^2$, 35 $mU/cm^2$, 40 $mU/cm^2$, 45 $mU/cm^2$, 50 $mU/cm^2$, 55 $mU/cm^2$, 60 $mU/cm^2$, 65 $mU/cm^2$, 70 $mU/cm^2$, 75 $mU/cm^2$, or 80 $mU/cm^2$. For example, the membrane is coated with heparin in an amount of from 5 $mU/cm^2$ to 25 $mU/cm^2$ of the membrane surface.

## Brief Description of the Drawings

Figure 1 is a schematic representation of sieving curves of hypothetic membranes having different selectivities as illustrated by their respective MWRO and MWCO values. When the difference between the molecular weight retention onset (MWRO) and molecular weight cut-off (MWCO) of a membrane decreases, the profile of the curve becomes steeper which corresponds to an increased selectivity. Starting from Membrane 1, this means that, for example, the MWCO can remain the same for both membranes (MWCO1 and MWCO2), while the MWRO is shifted to higher molecular weights, resulting in MWRO2 and in a Membrane 2 that has an increased selectivity. If the MWRO is shifted to higher molecular weights and the MWCO is shifted to lower molecular weights, the resulting Membrane 3 would have an even higher MWRO (MWRO3) combined with a lower MWCO (MWCO3), resulting in Membrane 3 having a further increased selectivity. It would also be possible to keep the MWRO3 at the same level as MWRO2 and only shift the MWCO3 to lower molecular weights (not shown here), which would also result in an improved selectivity versus Membrane 1. In consequence, methods for modulating the MWRO and MWCO of a given membrane mean that the removal of small, medium, and large middle molecules can be improved while the loss of albumin and other proteins in this molecular weight range and above can be decreased to achieve a better selectivity.

Figure 2 is a schematic representation of various processes that can be applied to modify a given membrane or membrane material with polydopamine and heparin for modifying its selectivity. Figure 2(0) shows a finished hollow fiber membrane ("base membrane") without any polydopamine (PD) and/or heparin (HEP) modification. Modification can be done successively by coating the hollow fiber membrane in a first step with polydopamine followed by coating with heparin in a second step (Figure 2(A)). Alternatively, the coating can be done in one step with a coating solution comprising both (poly)dopamine and heparin (Figure 2(B)). Another option is shown in Figure 2(C), where the membrane is modified in situ with polydopamine during spinning by adding it to the polymer. Heparin can then be coated onto the membrane in a second step (not shown).

Figure 3 is a schematic representation of how the coating of the invention is hypothesized to improve the selectivity of a membrane. Figure 3(A) shows an unmodified membrane. Negatively charged molecule such as, for example, albumin can just pass through a membrane pore of corresponding size. Figure 3(B) shows a membrane which is coated with polydopamine (PD) and heparin (HEP) whereby the membrane and pore receive a negatively charged layer and therefore reject passage of the same molecule which would have otherwise passed the pore. Another or additional effect that may contribute to an increase of rejection after modification is shown in Figure 3(C). By reducing the pore size of larger pores more than smaller ones the selectivity of the overall membrane can also be increased.

Figure 4 is a schematic representation of the process of coating hollow fiber membranes in a filter module, such as a minimodule, in recirculation mode, whereby the coating solution is pumped through the filter or minimodule until the intended coating is achieved.

Figure 5 depicts sieving coefficients of myoglobin and albumin in water and pure water permeance (PWP) at 37°C of commercial hollow fiber membranes (Revaclear, Baxter) either untreated (HF), coated with 0.5 g/L polydopamine (HF-PD), or coated with 0.5 g/L polydopamine and, subsequently, with 1 g/L heparin (HF-PD-HEP). It is shown that the sieving coefficient of myoglobin increases and reaches 100% with a full coating with polydopamine and heparin, whereas the sieving coefficient of albumin remains the same. In other words, the selectivity of the coated membrane increases.

Figure 6 shows hollow fibers with and without *in situ* modification with polydopamine. Membrane A is not modified with polydopamine. Membrane C is *in situ* modified with polydopamine in the polymer solution (0.5 wt.%, relative to the total weight of the polymer solution). Membrane E is also modified with polydopamine but has been prepared with a bore liquid (center fluid) comprising tris buffer. A slightly brownish color from polydopamine can be seen in Membrane E.

Figure 7 shows the morphology of a)-c) $HF_{is}$ with no dopamin, d)-f) $HF_{is}$-PD and g) $HF_{is}$-PD-HEP fibers: a)+d) cross-section; b)+e) pore structure of enlarged cross-section; c)+f)+g) lumen channel of enlarged cross-section.

Figure 8 depicts the results of zeta potential measurements with various membranes. Figure 8A shows $HF_{is}$ and $HF_{is}$-PD, as well as $HF_{is}$-HEP and $HF_{is}$-PD-HEP. Figure 8B shows membranes prepared with a center fluid comprising tris buffer, $HF_{is}$-PD-T and $HF_{is}$-PD-HEP-T. Binding of heparin and with it a significant decrease of the zeta potential is visible only where polydopamine has been effectively incorporated into the membrane. In situ incorporated polydopamine only does not have any relevant influence on the zeta potential of a membrane.

Figure 9 shows the heparin activity on the fiber lumen of hollow fibers taken from minimodules or filters that were coated with polydopamine and heparin in a one-step approach as described in Example 2. "n" refers to the number of samples that were prepared from a given device and tested for heparin activity. "MM" refers to "minimodule" that was submitted to the coating procedure, whereas "Filter" means that a hemodialyzer was subjected to the coating. Except for the Evodial membrane all membranes used were MCO Ci-400 membranes that were unsterile or sterilized with e-beam as indicated. "D6C9.5H0.5" refers to a MCO Ci-400 membrane coated with 6 mg/mL dopamine, 9.5 mg/mL chondroitin and 0.5 mg/mL heparin. "D12H0.5" refers to a MCO Ci-400 membrane coated with 12 mg/mL dopamine and 0.5 mg/mL heparin.

**Detailed Description**

The expression "selectivity" as used herein for a hemodialysis membrane refers to the membrane's ability to efficiently remove small (0.5-15kDa), medium (>15-25kDa) and large (>25-58kDa) middle-molecular weight compounds (see also Rosner et al.: Classification of Uremic Toxins and Their Role in Kidney Failure. Clin J Am Soc Nephrol. 2021) while essentially retaining larger proteins such as albumin (66.4 kDa). Such selectivity is described, for example, by the steepness of the sieving curve (e.g., dextran sieving curve) of a membrane. Increased steepness of the sieving curve correlates to increased selectivity. Accordingly, selectivity of a membrane can be described by its MWRO (molecular weight retention onset), i.e., the molecular weight at which the sieving coefficient of the membrane is 0.9, and its MWCO (molecular weight cut-off), i.e., the molecular weight at which the sieving coefficient of the membrane is 0.1, as these parameters provide for the necessary information on the shape of a membrane's sieving curve and, accordingly, on the above-mentioned removal and retention capabilities or selectivity of the membrane. For example, two membranes may have the same MWCO while differing in their MWRO. The membrane having the lower MWRO, accordingly, is less effective in removing molecules of a molecular weight below the cut-off. Its sieving curve is less steep, and the membrane is less selective than the one having a higher MWRO that is, accordingly, closer to the MWCO of the membrane, resulting in a better removal of said middle molecules while the retention capabilities remain the same.

The expression "MCO membrane(s)" or "MCO dialyzer(s)" as used herein, which is sometimes interchangeably used with the expression "HRO membrane(s)" or "HRO dialyzer(s)" for "High Retention Onset" membrane(s) or dialyzer(s), refers to membranes and dialyzers such as described, for example, in WO 2015/118045 A1 and WO 2015/118046 A1, respectively, and wherein the membrane(s) preferably has (have) a MWRO of from 9 to 12.5 kDa and a MWCO of from 55 to 110 kDa as measured by dextran sieving before blood contact and as otherwise described in WO 2015/118046 A1, and preferably further has (have) a sieving coefficient for albumin ($Sc_{Alb}$(%)) of below 1 (<1) as measured in human plasma at QB=300ml/min and QUF=60ml/min; a sieving coefficient for β-2-microglobulin ($Sc_{\beta 2m}$ (%)) of equal to or more than 95 (≥95) as measured in human plasma at QB=300ml/min and QUF=60ml/min; and a sieving coefficient for YKL-40 ($SC_{YKL-40}$ (%)) of equal to or more than 30 (≥30) as measured in human plasma at QB=300ml/min and QUF=60ml/min. An MCO dialyzer as mentioned herein is preferably further characterized by a KUF (ml/h/mmHg) of equal to or higher than 20 (≥20) as measured according to ISO 8637; a β2m clearance $K_{\beta 2m}$ (ml/min)of higher than 80 (>80) as measured

according to ISO 8637 with QB=300-400mL/min; QD=700mL/min; QUF=0-10mL/min; a YKL-40 clearance $K_{YKL-40}$ (ml/min) of equal to or higher than 20 (≥20) as determined in human plasma with QB=300ml/min; QD=500ml/min; QUF=10ml/min and preferably further has an albumin loss (g/4h in vivo treatment or simulated use correlated with in vivo use) of equal to or below 7 (≤7.0), more preferably of equal to or below 4 (≤4.0).

The expression "dopamine" as used herein refers to the compound 2-(3,4-dihydroxyphenyl)ethylamine of formula (I) and including commercially available forms thereof, such as, for example, dopamine-hydrochloride (2-(3,4-dihydroxyphenyl)ethylamine-hydrochloride).

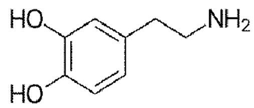

Formula (I)

The expression "polydopamine" (PD) refers to the polymer obtained from dopamine, for example by (auto)oxidation of dopamine in a basic medium (see, for example, Salomäki et al. J. Phys. Chem. B (2018), 122, 6314-6327. The expression "polydopamine" encompasses various stages of polymerization starting from dopamine and, accordingly, dopamine and polydopamine may exist in various ratios during such process of polymerization.

The expression "heparin" as used herein refers to linear, unbranched, and highly sulfated polysaccharides belonging to the family of glycosaminoglycans (GAGs) and including (i) heparin and heparan sulfate, and (ii) hyaluronan. The expression "heparin" encompasses the three forms of heparins which are available for therapeutic purposes, (i) unfractionated heparin (UFH), (ii) low molecular weight heparin (LMWH) and (iii) synthetic ultra (U)LMWH, see, for example, Laner-Plamberger et al., Int. J. Mol. Sci. 2021, 22, 12041.

The expression "base membrane" as used herein refers to a membrane before being modified or coated with polydopamine and heparin. In other words, the base membrane and the coated membrane only differ with regard to the presence of polydopamine and heparin but are otherwise the same. Regarding *in situ* polydopamine modified membranes, the expression "base membrane" refers to a membrane that is essentially prepared from the same polymer solution but does not contain polydopamine, and which is otherwise not coated with heparin.

It was a goal of the present invention to provide for a method for increasing the selectivity of a membrane by modifying them with polydopamine and heparin. The membranes surprisingly show an increased selectivity versus base membranes without such modification. In addition, they show an excellent hemocompatibility and antithrombogenicity. Such modification can be achieved in different ways according to the invention.

Membranes that can be used as base membranes according to the invention may be polysulfone (PSf) or polyethersulfone (PES) based membranes. They comprise a blend of i) a polysulfone, a polyethersulfone or a polyarylethersulfone and ii) polyvinylpyrrolidone. Base membranes according to the invention can also be made from other known materials as the concept of selectivity increase as described herein is connected to the modification of the pores of a given membrane. Other membrane materials that can be used are, for example, sulfonated polyacrylonitrile (PA or AN69), poly(methyl methacrylate) (PMMA), CTA (cellulose triacetate) or EVAL (ethylene vinyl alcohol copolymer). For an overview see, for example, Said et al., A Review of Commercial Developments and Recent Laboratory Research of dialyzers and Membranes for Hemodialysis Application. Membranes 11, 767 (2021) .

According to one aspect, base membranes according to the invention can have different physical structures, including varying degrees of asymmetric configuration, ranging from minimum asymmetry in sponge-like structures to maximum asymmetry in finger-like structures, such as described, for example, in Ronco and Clark, Nature Reviews. Nephrology 14 (6) (2018): 394-410). In case of hollow fiber membranes, the lumen or inner side of the hollow fibers membranes is preferably coated as it generally provides for the selective layer of the hollow fiber membranes that is in contact with blood, or, optionally, with blood plasma. In devices where the outer side of a hollow fiber membrane is in contact with blood and provides for the selective layer of the membrane, a modification or coating according to the invention would preferably be applied to the outer side of the hollow fiber membranes.

According to one aspect, the base membranes are MCO membranes. A representative of such MCO membrane which is available in the market today is the Theranova dialyzer (Baxter) which comprises such membrane.

According to another aspect, the base membranes comprise a blend of i) a polysulfone, a polyethersulfone or a polyarylethersulfone and ii) polyvinylpyrrolidone.

According to another aspect, the base membranes have a MWRO of from 8.5 to 14.0 kDa and a MWCO of from 50.0 to 130.0 kDa as measured by dextran sieving before blood contact, preferably a MWRO of from 8.5 to 12.5 kDa and a MWCO of from 55.0 to 110.0 kDa as measured by dextran sieving before blood contact. According to yet another aspect, the base membranes have a MWRO of from 9.0 to 12.5 kDa and a MWCO of from 55.0 to 90.0 kDa as measured by dextran sieving before blood contact.

According to another aspect, the base membranes have a MWRO of from 15kDa to 20kDa and a MWCO of from 170kDa to 320kDa as measured by dextran sieving before blood contact. Such membranes are described, for

example, in WO 2004/056460 A1 or in Gondouin and Hutchison: High Cut-Off Dialysis Membranes: Current Uses and Future Potential. Advances in Chronic Kidney Disease 18 (2011):180-187. A representative of such HCO membranes available in the market today is the Theralite dialyzer (Baxter) which comprises such membrane.

According to yet another aspect, the base membrane is a high-flux (HF) membrane having a MWRO of from 5.0 kDa to 8.5 kDa and a MWCO of from 25 kDa to 50 kDa as measured by dextran sieving before blood contact.

According to one aspect of the invention, the membranes can be coated in one step with a coating solution comprising polydopamine and heparin **(Figure 2B**). For the avoidance of doubt, dopamine is the starting material which is initially provided in the solution for coating the base membrane. It reacts to polydopamine in the solution and is applied together with heparin to the membrane surface. Accordingly, as used herein, dopamine refers to the starting material added to the coating solution but implies that it is transferred into polydopamine in the process of coating and is essentially present as polydopamine on the final membrane.

According to one aspect, the solution for coating the base membrane is prepared, for example, by providing a dopamine solution in a buffer, such as, for example, tris buffer, at an elevated pH to start the polymerization of dopamine. According to one aspect, the pH is from about 8.0 to 10.0, such as from 8.0 to 9.8, from 8.0 to 9.6, or from 8.2 to 9.4. A color change is indicative for polydopamine formation. Heparin is then added to the solution and dissolved therein. The solution can then be applied to, for example, the lumen of a hollow fiber membrane for the coating of the lumen of the membrane. For example, the coating solution can be filled into the lumen of the fibers by aspiration. The hollow fiber membrane can be incubated with the coating solution for a certain time which can be varied from several minutes to several hours. Generally, ten minutes to two hours of incubation time will be sufficient depending on the base membrane and the concentration of the dopamine and heparin in the solution. The lumen of the hollow fiber can then be rinsed, e.g., with 0.9% NaCl. The flow rate during rinsing can be varied over a relatively broad range, e.g., from about 10 mL/min to 50 ml/min, and the time can also be varied from about 1 to 20 minutes, such as, for example, from about 2 to 10 minutes.

According to another aspect, the coating solution can be applied to the outer surface of the hollow fiber depending on the intended use of the membrane and device comprising same. The coating solution can also be applied to the surface of a flat sheet membrane.

According to another aspect, hollow fiber membranes can be coated in recirculation mode, wherein the coating solution is provided in a pool from which it is pumped, for example, through the hollow fibers of a filter device **(Figure 4**). The pool volume can vary, but about 80 to 500 mL of a coating solution will be sufficient for an effective coating of a standard dialyzer having a surface area of about 1.5 -2.2 $m^2$. The pump flow can also be varied over a relatively broad range, such as, for example, from 1 mL/min to about 20 mL/min. After coating, the filters can be rinsed as described before.

The process provides for a coated membrane comprising or essentially consisting of a base membrane which is coated with one essentially homogenous coating layer which comprises or essentially consists of a mixture of polydopamine and heparin.

According to one aspect, the concentration of dopamine in the coating solution is between 2 mg/mL to 80 mg/mL. According to another aspect of the invention the concentration of dopamine in the coating solution is between 3 mg/mL and 60 mg/mL. According to yet another aspect of the invention the concentration of dopamine in the coating solution is from 5 mg/mL to 25 mg/mL. According to yet another aspect of the invention the concentration of chondroitin in the coating solution is between 5 mg/mL and 80 mg/mL, such as between 10 mg/mL and 60 mg/mL.

According to another aspect, the concentration of heparin in the coating solution is between 0.1 mg/mL to 100 mg/mL, from 0.5 mg/mL to 80.0 mg/mL, from 1 mg/mL to 60 mg/L, or from 5 mg/mL to 40 mg/L. For example, the concentration of heparin in the coating solution is 0.5 mg/mL, 1 mg/mL, 2 mg/mL, 3 mg/mL, 4 mg/mL, 5 mg/ml, 10 mg/ml, 15 mg/ml, 20 mg/ml, 25 mg/ml, 30 mg/ml, 35 mg/ml, 40 mg/ml, 45 mg/ml, 50 mg/ml, 55 mg/ml, 60 mg/ml, 65 mg/ml, 70 mg/ml, 75 mg/ml, or 80 mg/ml. According to a further aspect, the concentration of heparin in the coating solution is between 10 mg/mL and 50 mg/mL. According to a further aspect, the concentration of heparin in the coating solution is between 0.5 mg/mL to 20 mg/mL or from 0.1 mg/mL to 10 mg/mL.

According to another aspect, the membranes are coated with polydopamine in an amount of from 0.30 $\mu g/cm^2$ to 1.10 $\mu g/cm^2$ of the membrane surface. According to another aspect of the invention, the membranes are coated with chondroitin in an amount of from 0.50 $\mu g/cm^2$ to 1.20 $\mu g/cm^2$, such as 0.30 $\mu g/cm^2$, 0.40 $\mu g/cm^2$, 0.50 $\mu g/cm^2$, 0.60 $\mu g/cm^2$, 0.70 $\mu g/cm^2$, 0.80 $\mu g/cm^2$, 0.90 $\mu g/cm^2$, 1.00 $\mu g/cm^2$, 1.10 $\mu g/cm^2$, or 1.20 $\mu g/cm^2$. According to yet another aspect of the invention, the membranes are coated with polydopamine in an amount of from 0.40 $\mu g/cm^2$ to 0.60 $\mu g/cm^2$ of the membrane surface. According to yet another aspect of the invention, the membranes are coated with chondroitin in an amount of from 0.30 $\mu g/cm^2$ to 0.70 $\mu g/cm^2$.

According to another aspect, the membrane is coated with heparin in an amount of from 5 $mU/cm^2$ to 100 $mU/cm^2$ of the membrane surface, from 5 $mU/cm^2$ to 80 $mU/cm^2$ of the membrane surface, from 5 $mU/cm^2$ to 50 $mU/cm^2$ of the membrane surface, or from 5 $mU/cm^2$ to 30 $mU/cm^2$ of the membrane surface, such as, for example 5 $mU/cm^2$, 6 $mU/cm^2$, 7 $mU/cm^2$, 8 $mU/cm^2$, 9 $mU/cm^2$, 10 $mU/cm^2$,

11 $mU/cm^2$, 12 $mU/cm^2$, 13 $mU/cm^2$, 14 $mU/cm^2$, 15 $mU/cm^2$, 16 $mU/cm^2$, 17 $mU/cm^2$, 18 $mU/cm^2$, 19 $mU/cm^2$, 20 $mU/cm^2$, 21 $mU/cm^2$, 22 $mU/cm^2$, 23 $mU/cm^2$, 24 $mU/cm^2$, 25 $mU/cm^2$, 30 $mU/cm^2$, 35 $mU/cm^2$, 40 $mU/cm^2$, 45

mU/cm$^2$, 50 mU/cm$^2$, 55 mU/cm$^2$, 60 mU/cm$^2$, 65 mU/cm$^2$, 70 mU/cm$^2$, 75 mU/cm$^2$, or 80 mU/cm$^2$. For example, the membrane is coated with heparin in an amount of from 5 mU/cm$^2$ to 25 mU/cm$^2$ of the membrane surface.

The use of heparin for coating a membrane according to the invention reduces the thrombogenicity of the membranes **(Examples 6 and 7**). It was an important and surprising finding that the use of heparin in combination with polydopamine leads to an increased selectivity of the treated base membrane. At the same time, heparin can still impart an antithrombogenic function when coated onto said membrane in combination with polydopamine. Both the antithrombogenic effect and the selectivity increase are maintained also after sterilization.

According to another aspect, the base membranes are coated with polydopamine and heparin in two consecutive steps **(Figure 2A**), wherein the membranes in a first step are coated with polydopamine as described before for the mixture of polydopamine and heparin, followed in a second step by coating with heparin. Said process provides for coated membranes comprising or consisting of a base membrane which is coated with a first coating layer comprising or essentially consisting of polydopamine, a second essentially homogenous coating layer comprising or essentially consisting of heparin.

According to another aspect, the membranes are modified with polydopamine and heparin by *in situ* modification of the base membrane with polydopamine in a first step **(Figure 2C**), followed by coating of the polydopamine modified base membrane with heparin in a second step as described above. In situ modification with polydopamine can be achieved by directly including dopamine into the spinning solution as described in **Example 2.3.** The concentration of dopamine in the spinning solution can be varied over a certain range, such as, for example, from about 0.3 to 0.8 wt.%, such as from about 0.4 wt.% to 0.6 wt.%. Dopamine can either be added to the spinning solution after all ingredients except dopamine have been mixed until homogeneity or can immediately be added to the other ingredients for preparing the spinning solution. Polydopamine is formed upon spinning within the membrane of which it is then an integral part. The finished, polydopamine modified membrane can then be coated with heparin.

Polydopamine can also be coated on or added to a flat sheet base membrane according to any of the above processes, i.e., either by an *in situ* casting process or by a sequential coating with polydopamine, followed by heparin coating, or by a one-step coating with a mixture of polydopamine and heparin.

The presence and amount of polydopamine and heparin on the finished fibers can be tested with known assays such as a protein assay based on bicinchoninic acid for polydopamine (Smith et al., Analytical Biochemistry (1985) 150: 76-85).

The membranes that have been modified or coated according to the invention, and the filters comprising same, can be sterilized either with steam sterilization, e-beam sterilization at a dose of from about 25 to 30 kGy or gamma sterilization at about 25 to 30 kGy. Gamma sterilization can be done after drying with pressurized air after coating and filling with 2 grams of residual water prior to sterilization or with water filled filter devices, optionally in the presence of an oxygen scavenger. Alternatively, e-beam sterilization can be used for sterilization which well maintains the integrity and stability of the coating and the performance of the coated hollow fibers and filter device. According to one aspect, e-beam and gamma ray sterilization is used in connection with modified and/or coated membranes according to the invention. According to yet another aspect, e-beam sterilization is used in connection with modified and/or coated membranes according to the invention.

According to one aspect of the invention, the above-described modification and/or coating of a given membrane, such as a polysulfone or polyethersulfone based membrane, with polydopamine and heparin has an unexpected influence on the sieving performance and specifically and in consequence, on the selectivity of the membrane. Marker compounds that can be used for assessing said influence by determining their respective sieving coefficients are advantageously selected over a range of different molecular weight and include, for example, compounds representative for small middle molecules, medium middle molecules, large middle molecules and large molecules as defined above. Examples are, accordingly, β2-M, myoglobin, complement factor D, YKL-40 and albumin. For determining the effect of the coating according to the invention on the sieving performance and selectivity of a given base membrane, sieving coefficients for selected marker molecules are determined before and after the modification or coating with polydopamine and heparin. Said sieving coefficients can then be compared. As explained on the basis of MWRO and MWCO in **Figure 1**, a similar assessment can be made based on, for example, the sieving coefficients of the molecules with a lower and a higher molecular weight, respectively. For example, maintaining the sieving coefficient of a lower molecular weight molecule, such as, for example, YKL-40, while at the same time the sieving coefficient for albumin can be decreased, indicates an increase in selectivity as the membrane gets less permeable for albumin whereas it retains its desired permeability for larger middle molecules. Comparisons of uncoated or unmodified membranes and membranes coated with polydopamine and heparin are provided in **Example 4, Tables II** and **III** for different membranes.

According to another aspect of the invention, the modified or coated membranes according to the invention are highly hemocompatible and they maintain their hemocompatibility also after sterilization especially in case of e-beam sterilization. According to one aspect of the invention, the modified and/or coated membranes can be used as membranes in medical applications, including for applications that require a direct contact of the coated surface with the blood or blood components of a patient. For example, modified and/or coated membranes according to the invention can be used

for preparing dialyzers for use in hemodialysis, including chronic and acute (CRRT) applications and encompassing hemodiafiltration and hemofiltration.

According to yet another aspect of the invention, filters or hemodialyzers are provided that comprise hollow fiber membranes that have been modified and/or coated according to the invention on the lumen side or on the outer surface of the hollow fiber membranes. According to one aspect, said filters or hemodialyzers comprise hollow fiber membranes which are coated with polydopamine and heparin. According to another aspect, said filters or hemodialyzers comprise hollow fiber membranes wherein the membranes are modified *in situ* with polydopamine and are coated with heparin.

According to another aspect of the invention, the filters and dialyzers of the invention that comprise hollow fiber membranes which are modified and/or coated with polydopamine and heparin can be used in extracorporeal blood purification or extracorporeal blood treatment, including, for example, hemodialysis, hemodiafiltration and hemofiltration.

It will be readily apparent to one skilled in the art that various substitutions and modifications may be made to the invention disclosed herein without departing from the scope and spirit of the invention.

The present invention will now be illustrated by way of non-limiting examples to further facilitate the understanding of the invention.

## Examples

### Example 1: Basic Materials and Methods

### Example 1.1: Preparation of minimodules

Minimodules (fibers in a housing used as standardized filters for analysis) are prepared by cutting fibers to a length of 20 cm, drying the fibers for 1 h at 40°C and <100 mbar and subsequently transferring the fibers into the housing. The ends of the fibers are potted with polyurethane. After the polyurethane has hardened, the ends of the potted membrane bundle are cut to reopen the fibers. The minimodule ensures protection of the fibers. In the present examples, minimodules having an effective membrane (lumen) surface A of 360 $cm^2$ were used. The number of fibers required for an effective surface A of 360 $cm^2$ is calculated according to equation (1)

$$A=\pi \times d_i \times l \times n\,[cm2] \quad (1),$$

wherein di is the inner diameter of fiber [cm], n represents the number of fibers, and l represents the effective fiber length [cm]. Each minimodule has an effective length of approx. 170 mm (without PU potting) and an inner diameter of 10 mm. The internal diameter of fibers and the wall thickness depends on the specific membranes used. Hence, the packing density generally varies between 23% and 31%.

### Example 1.2: Hydraulic Permeability (Lp) of minimodules

The hydraulic permeability of a minimodule provided in is determined by pressing a defined volume of water under pressure through the minimodule, which has been sealed on one side, and measuring the required time. The hydraulic permeability is calculated from the determined time t, the effective membrane surface area A, the applied pressure p and the volume of water pressed through the membrane V, according to equation (2):

$$Lp = V / [p \cdot A \cdot t] \quad (2)$$

The effective membrane surface area A is calculated from the fiber length and the inner diameter of the fiber according to equation (3)

$$A = \pi \cdot d_i \cdot l \cdot [cm^2] \quad (3)$$

wherein di represents the inner diameter of fiber [cm] and l represents the effective fiber length [cm].

The mini-module is wetted thirty minutes before the Lp-test is performed. For this purpose, the minimodule is put in a box containing 500 mL of ultrapure water. After 30 minutes, the minimodule is transferred into the testing system. The testing system consists of a water bath that is maintained at 37°C and a device where the minimodule can be mounted. The filling height of the water bath has to ensure that the minimodule is located underneath the water surface in the designated device. In order to avoid that a leakage of the membrane leads to a wrong test result, an integrity test of the minimodule and the test system is carried out in advance. The integrity test is performed by pressing air through the minimodule that is closed on one side. Air bubbles indicate a leakage of the minimodule or the test device. It must be checked if the leakage is due to an incorrect mounting of the minimodule in the test device or if the membrane leaks. The minimodule must be discarded if a leakage of the membrane is detected. The pressure applied in the integrity test must be at least the same value as the pressure applied during the determination of the hydraulic permeability in order to ensure that no leakage can occur during the measurement of the hydraulic permeability because the pressure applied is too high.

**Example 1.3: Dextran sieving measurements**

**Example 1.3.1: Dextran solutions**

Fractions of dextran supplied by Fluka (Mw 6, 15-20, 40, 70, 100, 200, 500 kDa) and Sigma-Aldrich (Mw 9-11 kDa) (both from Sigma-Aldrich Co. LLC, St. Louis, USA) are used without further purification. Solutions of dextrans with the different molecular weight fractions are combined in Millipore water (i.e., Type 1 ultrapure water, as defined by ISO 3696) at a concentration of 1 g/l for each fraction, which results in an overall concentration of 8 g/l.

**Example 1.3.2: Dextran sieving coefficient tests**

Filtration experiments were carried out under a constant shear rate ($\gamma$=750s$^{-1}$) and with the ultrafiltration rate set at 20% of the blood side entrance flux $Q_{Bin}$, calculated according to equation (4):

$$Q_{Bin} = \frac{\gamma \cdot n \cdot \pi \cdot d_i^3 \cdot 60}{32} \quad (4),$$

where $Q_{Bin}$ is the flux at the blood side entrance in ml/min; n is the number of fibers in the minimodule; $d_i$ is the inner diameter of the fibers in cm and $\gamma$ is the constant shear rate mentioned above. The filtration conditions are without backfiltration, contrary to the conditions typical of hemodialysis. The dextran solution was recirculated at 37°C ± 1°C. Feed (blood side entrance), retentate (blood side exit), and filtrate (dialysate exit) samples were taken after 15 min. Relative concentrations and molecular weights of the samples were analyzed via gel permeation chromatography. The analysis was carried out in a High Performance Liquid Chromatography (HPLC) device (HP 1090A or Agilent 1200; Agilent, Santa Clara, CA, USA) equipped with an RI detector (G1362 from Agilent) and TSKgel columns (PWXL-Guard Column, G 3000 PWXL, G 4000 PWXL; Tosoh, Tessenderlo, Belgium). Samples were filtered through a 0.45 μm filter type OE67 from Schleicher and Schnell, Einbeck, Germany. Calibration was done against dextran standards (Fluka). The sieving coefficient SC is calculated according to equation (5)

$$SC = \frac{2 \cdot c_F}{c_p + c_R} \quad (5),$$

wherein $c_F$ is the concentration of the solute in the filtrate, $c_P$ its concentration in the permeate and $c_R$ its concentration in the retentate.

**Example 1.4: Measurement of sieving coefficients in human plasma**

Middle molecules, consisting mostly of peptides and small proteins with molecular weights in the range of 500-60,000 Da, accumulate in renal failure and contribute to the uremic toxic state. Beta2-microglobulin (beta2-MG or β2-M) with a molecular weight of 11 kDa is considered one of the smaller representatives of these middle molecules.

Myoglobin has a molecular weight (MW) of about 17 kDa is already larger. It will not be completely cleared from blood by known high-flux dialyzers, whereas it is readily removed by high cut-off dialyzers. Complement factor D is a serine protease with a molecular weight of 25 kDa and is a good candidate for assessing the sieving coefficient for a molecule in the medium molecular weight range. YKL-40 with a molecular weight of 40 kDa is considered a representative of higher molecular weight middle molecules that should still be removed as efficient as possible. Albumin with a MW of about 67 kDa is a key element in describing the sieving characteristics of membranes, as albumin should preferably not be allowed to pass a membrane for chronic hemodialysis to a significant extent, whereas molecules having a lower molecular weight, such as β2-M and YKL-40 should be removed as efficiently as possible, i.e., the sieving curve of a given membrane should be steep and not flat, as is typically found with protein-leaking membranes.

The sieving coefficients for albumin and YKL-40 which are naturally contained in human plasma can be determined for uncoated membranes and membranes that were coated according to the invention or, for comparison, coated with dopamine only. Determination of sieving coefficients for these marker molecules was done with Octaplas LG from Octapharma. The respective mini-modules as described in **Example 1.1** and coated as described in **Example 2** and in **Example 4** were used.

For the experiments, human plasma was thawed and brought to 37°C under careful stirring. Other marker substances can then be added to the plasma as required. For each minimodule 150g of the human plasma was used. The final protein concentration of the test solution was 45±5 g/l. The minimodules were rinsed with 40 mL 0.9% NaCl solution on the blood and dialysate side. Then the blood and dialysate sides are blown out at 0.3 ± 0.1 bar for 10 ± 5 s. $Q_B$ was 8,7 mL/min (= 7,71 SKT) and $Q_{UF}$ was 1,7 mL/min (=1,58 SKT). The minimodules were connected to a new tubing set with the open filtrate side on top. The recirculating tubes were returned to the solution reservoir. The blood-in pump was started. When the minimodules were free of air bubbles, the filtrate pump was started. As soon as filtrate flows back from the tube into the pool time was started. After 30 minutes test time a sample of the test solution (A) (minimum 1,1 mL for HSA and 550 μL for YKL-40) was taken. The sampling time was 1 min for $Q_{UF}$ and 1 min for $Q_{Bout}$. From these measurements, the retentate sample (R) and filtrate sample (F) are used for further analysis of the marker molecules. The sieving coefficient SC is calculated according to equation (5).

**Example 1.6: Clearance Performance**

The clearance "C" (mL/min) refers to the volume of a solution from which a solute is completely removed per unit time. In contrast to the sieving coefficient which is the best way to describe a membrane as the essential component of a hemodialyzer, clearance is a measure of the overall dialyzer function and hence dialysis effectiveness. Accordingly, the coating as described before was applied to complete hemodialyzers, such as commercially available dialyzers like Theranova 400 (Baxter Int., Inc.) or hemodialyzers were prepared from other MCO type membranes, such as the MCO-Ci 400 dialyzer (Gambro Dialysatoren GmbH, Hechingen, Germany) or the MCO 3 prototype (Gambro Dialysatoren GmbH, Hechingen Germany; see, for example, Boschetti-de-Fierro et al. (2015), Scientific Reports 5: 18448, DOI: 10.1038/srep18448) and were coated according to the invention. If not indicated otherwise, the clearance performance of a dialyzer was determined according to ISO 8637:2004(E). The set-up of the test circuit was as shown in ISO 8637:2004(E). Flows are operated in single path.

To measure clearance, human plasma (protein content 55 ± 10 g/L) is spiked with myoglobin and β2-M (Lee Biosolutions), to a concentration of 0.50 mg/L and 5.0 mg/L , respectively. Albumin, complement factor D and YKL-40 are measured directly from plasma concentrations. Samples are in each case taken from the blood outlet, dialysate outlet and blood inlet, respectively. A first sample is taken after 4 minutes.

**Example 1.7: Quantitative determination of YKL-40 and complement factor D in human plasma**

For determining the concentration of YKL-40 and human complement factor D the Quantikine Human Chitinase 3-like (CHI3L1) and Quantikine ELISA Human Complement Factor D (CFD) ELISA Immunoassay (R&D Systems Europe, Ltd.), respectively, are used according to the supplier's instructions. The assays are a 4.5- and 3.5-hour solid phase ELISA designed to measure CHI3L1 and CFD in cell culture supernates, serum, plasma, and urine. Both assays employ the quantitative sandwich enzyme immunoassay technique. A monoclonal antibody specific for the analyte has been pre-coated onto a microplate. Standards and samples are pipetted into the wells and any analyte present is bound by the immobilized antibody. After washing away any unbound substances, an enzyme-linked polyclonal antibody specific for the analyte is added to the wells. Following a wash to remove any unbound antibody-enzyme reagent, a substrate solution is added to the wells and color develops in proportion to the amount of CHI3L1 or CFD bound in the initial step. The color development is stopped, and the intensity of the color is measured.

For determining the concentration of β2-M, myoglobin and albumin a nephelometer (BN ProSpec, Siemens Medical Solutions) is used in combination with the supplier's quantification kits. β2-M, myoglobin and albumin are quantified using the N Latex β2 Microglobulin kit, N Latex Myoglobin kit and N Antiserum to Human albumin kit (Siemens

Health solutions), respectively, all according to the supplier's instructions. Each determinant is measured separately by formation of an insoluble antigen-antibody complex upon addition of the provided antibody. The complex formation proceeds in excess of antibody, is measured using light scattering by the nephelometer and compared to standards of known concentration.

**Example 2: Coating of hollow fiber membranes with polydopamine and heparin**

**Example 2.1: One-step coating with polydopamine and heparin**

A 6 mg/mL or 12 mg/mL dopamine solution (600 mg dopamine hydrochloride from Sigma Aldrich in 100 mL of tris buffer (10 mmol/L, pH 8.6)) was prepared and the solution was stirred for 30 minutes to start the polymerization of dopamine. A color change is indicative for polydopamine formation. Then, 20 mg/mL or 40 mg/mL of heparin were added and the solution was stirred for about 5 minutes. The resulting solution was immediately used for a one-step coating by filling the lumen of the fibers of a minimodule from bottom to top by aspiration with a syringe. During filling the minimodule was shaken to remove air bubbles. The minimodule was then incubated for 1 hour at room temperature before the solution was removed. The minimodule was rinsed using 0.9% NaCl at a flowrate of 20 mL/min for 5 min on the lumen-side, followed by 5 min on the filtrate-side, followed by the repeat with Millipore water. After rinsing the minimodule was dried using compressed air for 80 minutes at 0,3 bar.

Alternatively, coating of filters or minimodules can be done in recirculation mode as shown schematically in **Figure 4,** which reduces the time required for coating compared to the static approach described above. In this case, minimodules or filters are coated on the lumen side of the fibers with a pump flow of, for example, 5 mL/min and a pool volume of about 100 mL for coating an average of four minimodules (25 mL per minimodule). During recirculation, the filtrate side of the minimodules or filters were closed with a connector. Concentrations of dopamine and heparin are as indicated above and/or in the respective Examples.

The minimodules, after coating, were tested the next day or sterilized first either with steam sterilization, e-beam sterilization at a dose of from about 25 to 30 kGy or gamma sterilization at about 25 to 30 kGy before testing. Gamma sterilization can be done after drying with pressurized air after coating and filling with 2 grams of residual water prior to sterilization or with water filled minimodules (or filters), optionally in the presence of an oxygen scavenger. Alternatively, e-beam sterilization can be used to maintain the integrity and stability of the coating and the performance of the coated hollow fibers and filter device.

**Example 2.2: Sequential (successive) coating with polydopamine and heparin**

For a sequential coating of the hollow fiber membranes the same minimodules and solutions were used. However, the dopamine solution was applied to the lumen of the hollow fibers once the dopamine had started to polymerize without adding heparin, followed by incubation for 1 hour and subsequent rinsing and flushing as described above. In a second step, the polydopamine-coated membrane was submitted to coating with heparin, which was applied to the hollow fibers as described above, followed by incubation for another hour and subsequent rinsing and flushing. The coated minimodules were set aside for testing on the next day.

For comparison, minimodules were also used for coating with either polydopamine with or without heparin as described above. The goal was to determine the respective influence of polydopamine alone and polydopamine in combination with heparin on the selectivity of a given membrane in comparison to an untreated base membrane.

**Example 2.3: In situ modification with polydopamine**

Instead of modifying or functionalizing membranes such as described herein post-production by coating with polydopamine and heparin, they can also be modified with polydopamine *in situ,* followed by coating with heparin. For the membrane production, polyethersulfone (PES) (Ultrason 6020 P, BASF, Germany), polyvinylpyrrolidone K90 (PVP K90) (average molecular weight of 360 kDa, Carl Roth, Germany) and polyvinylpyrrolidone K30 (PVP K30) (average molecular weight of 40 kDa, Carl Roth, Germany) are purchased. N-methyl-2-pyrrolidone (NMP) (99 %, Fisher Scientific, Germany) functions as solvent for the membrane preparation. Dopamine hydrochloride (98 %, Sigma Aldrich, Germany) was used as functional ingredient. For the membrane post-treatment, glycerol was purchased at Carl Roth with a purity above 99 %. Tris-(hydroxymethyl)-aminomethan (tris) (≥99.9 %, Carl Roth, Germany), sodium acetate (≥99 %, Carl Roth, Germany) and hydrochloric acid (ACS reagent, Sigma Aldrich, Germany) were used as buffer solutions and to adjust the pH of the different solutions.

For the modification of membranes with polydopamine, solutions were prepared and used in different membrane functionalization procedures to achieve the desired membrane structures. The polymer solution is summarized in **Table 0:**

***Table 0*: *Overview of polymer solution compositions for flat sheet casting and hollow fiber spinning***

| Polymer Solution | PES [wt.%] | NMP [wt.%] | PVP K30 [wt.%] | PVP K90 [wt.%] | Dopamine [wt.%] |
|---|---|---|---|---|---|
| P1 | 22.0 | 78.0 | - | - | - |
| P2 | 21.9 | 77.6 | - | - | 0.5 |
| P3 | 15.9 | 75.6 | 4 | 4 | 0.5 |
| **P4** | **14.0** | **84.0** | **-** | **2** | **-** |
| **P5** | **14.0** | **83.5** | **-** | **2** | **0.5** |
| P6 | 14.0 | 85.5 | - | - | 0.5 |

For the polymer solutions P1 to P4, all ingredients were stirred with a KPG stirrer at room temperature for at least 24 h until a homogeneous solution was formed. Before membrane fabrication, the solutions were left for degassing overnight to avoid defects in the membranes. For the polymer solutions P5 and P6, all ingredients except dopamine were stirred with a KPG stirrer at 65°C for at least 24 h until a homogeneous solution was formed. 2 h before transforming the polymer solutions into membranes, dopamine was added in an amount of 0.5 wt.% into the polymer solution and stirred until a homogeneous solution was formed. Subsequently, the solutions were left degassing around 2 h. The polymer solution turns brownish after adding dopamine due to polymerization.

The bore solution consisted of NMP (50 wt.%) and $H_2O$ (wt.%), herein referred to as **B1.** Alternatively, Tris buffer (10 mM, 50 wt.%, herein referred to as **B2**) can be used instead of $H_2O$ which promotes the polymerization of dopamine to polydopamine directly on the selective layer of the membrane. For fiber fabrication, a conventional spinning setup is used. The spinning parameters were as follows: Polymer solution flow rate [g/min] was 6.8, the polymer solution temperature [°C] was 65°. The Bore Flow rate [mL/min) was 10.3. The air gap was 2cm and the coagulation bath temperature was 65°C. During spinning, the polymer and the bore solution passed through a double orifice spinneret with a diameter of 0.4 mm for the bore and 1.12 mm for the polymer solution. The fiber emerged from the spinneret, passed through the air gap and entered the coagulation bath. The coagulation bath consisted of DI water. The take-up speed was controlled by a motorized pulling wheel, which conveys the fiber into a washing bath. Then, the fibers were stored in DI water for 34 h before storing them for another 24 h in a 5 wt.% glycerol in DI water solution. Finally, the fibers are air dried at room temperature overnight before further use.

Accordingly, various in situ polydopamine modified membranes were prepared and heparin was immobilized in a second step by filling the follow fibers in a membrane module with a heparin coating solution containing 1 mg/mL heparin and on the shell side sodium acetate buffer. After 24 h the hollow fiber membranes were flushed with DI water on the lumen and shell side. The resulting membranes were examined further. **Table I** shows which membranes have been prepared. The membranes slightly differ in their polymer composition (see also **Table 0**), bore solution used and their coating with heparin.

**Figure 6** shows the fibers of Membranes A , C and E. The single fibers do not differ significantly in color even though polydopamine was present in the polymer solution of fibers C and E in an amount of 0.5 wt.%. Only a slight brownish discoloration of Membrane E, $HF_{is}$-PD-T, suggests the polydopamine in the membrane structure. The morphology of Membranes A, $HF_{is}$, C, $HF_{is}$-PD and E, $HF_{is}$-PD-T does not show any significant differences either **(Figure 7**). Also, a subsequent coating with heparin on the lumen side of Membrane A ($HF_{is}$), resulting in Membrane B ($HF_{is}$-HEP), shows no visible change in the selective layer **(Figure 7 g)**). The surface of the lumen channel of the enlarged cross-section of Membrane A ($HF_{is}$) seems to be only marginally rougher than Membrane C ($HF_{is}$-PD). Furthermore, the polydopamine within the polymer solution only marginally influences the formation of the pore structure (**Figure 7 b) and e)**).

***Table I:*** *Overview over in situ modified hollow fiber membranes with ($HF_{is}$-PD) and without ($HF_{is}$-PD-HEP) heparin functionalization. "T" indicates that tris buffer was used as bore solution.*

| Membrane Type | | Polymer Solution/ Bore Solution | Heparin coating solution [mg/mL] | Coagulation Bath Temperature [°C] |
|---|---|---|---|---|
| A | $HF_{is}$ | P4/B1 | - | 65 |
| B | $HF_{is}$-HEP | P4/B1 | 1 | 65 |
| C | $HF_{is}$-PD | P5/B1 | - | 65 |
| D | $HF_{is}$-PD-HEP | P5/B1 | 1 | 65 |

(continued)

| Membrane Type | | Polymer Solution/ Bore Solution | Heparin coating solution [mg/mL] | Coagulation Bath Temperature [°C] |
|---|---|---|---|---|
| E | $HF_{is}$-PD-T | P5/B2 | - | 65 |
| F | $HF_{is}$-PD-HEP-T | P5/B2 | 1 | 65 |

For further testing, one fiber per membrane type is glued into a 7 cm long tube by filling the whole permeate side with glue to counteract permeation during the measurement. With 5 L of 1 mM KCl solution, the membrane module is flushed before the measurement with a SurPASS electrokinetic analyzer is started. This analyzer automatically changes the pH of 3 to 6 by titration 0.1 M HCl and 0.1 M NaOH **(Figure 8**). For each measured pH value, the device measures the zeta potential of the surface three times. Like that information can be gained on the charge and charge changes of the membrane surfaces. The zeta potential of Membrane A ($HF_{is}$) is negatively charged between pH 3 and 6. When polydopamine is added to the polymer solution ($HF_{is}$-PD), the charge of the lumen does not significantly change compared to that of Membrane A ($HF_{is}$), which suggests no or a very little amount of polydopamine on the membrane surface **(Figure 8A).** However, if these membranes are compared to the polydopamine-modified membrane after heparin coating, a clear difference in charge can be observed. It is assumed that the higher the charge difference of the membrane surface, the more heparin is present on the surface. Accordingly, the zeta potential can be used to qualitatively determine a coating with heparin. Even if there is no charge difference between $HF_{is}$ and $HF_{is}$-PD, the binding of heparin indicates the presence of polydopamine in the in situ polydopamine-modified membrane $HF_{is}$-PD-HEP. The presence of tris buffer in the bore solution also leads to a different zeta potential course **(Figure 8B).** Between pH 3 and 6 , the drop of the zeta potential is significantly more pronounced. The tris buffer in the bore solution influences the phase inversion process which in turn slightly changes the morphology of the membrane surface. **Figure 7(b)** shows the slight influence of tris buffer in the bore solution ($HF_{is}$-PD-T) compared to $HF_{is}$-PD **(Figure 7(a)**). Again, the binding of heparin shows another change in surface charge, indicating that heparin was bound on the membrane surface and has further lowered the zeta potential.

**Example 3: Quantification of polydopamine and heparin on membrane fibers**

The amount of polydopamine and of heparin on polyethersulfone (PES) fibers after coating were determined by placing fibers in 2 mL cryovials and adding the respective detection reagents for each substance.

Quantification of heparin can be done by various known methods, such as described in Lammers et al. A comparison of seven methods to analyze heparin in biomaterials: quantification, location, and anticoagulant activity. Tissue Eng Part C Methods. 2011, 17(6):669-76. For example, heparin was quantified with an anti-Xa chromogenic method, which is a one stage assay based on the inhibition of a constant amount and in excess of Factor Xa (FXa), by heparin to be assayed, in the presence of endogenous antithrombin (AT). The residual FXa hydrolyses a specific chromogenic substrate (SXa-11) releasing paranitroaniline (pNA). The amount of pNA released (measured by absorbance at 405 nm) is inversely proportional to the concentration of heparin (or other anti-Xa substance) present in the reaction medium.

For the quantification of polydopamine, a known protein assay based on bicinchoninic acid was used (Smith et al., Analytical Biochemistry (1985) 150: 76-85).

Both methods were utilized using a 200 pg/mL-1 stock solution, of which a defined amount was combined with 2mL of reagent. Since solid membranes were used, the dilution of the stock solution in the reagent was utilized to calculate the final standard concentration and this number was then used to determine the respective quantities on membrane the fibers. In this experiment, hollow fiber membranes based on polyethersulfone were coated with a solution of dopamine and heparin with concentrations of 6 mg/mL P5/B2dopamine and 20 mg/mL heparin. The samples were prepared in line with **Example 2.1** and the presence of polydopamine and heparin on the final membrane was determined.

Generally, polydopamine quantities were found to be in the range of from 0.37 to 0.63 $\mu g/cm^2$ of the membrane surface when coated with a solution as used herein. Heparin quantities were in the range of from 5 $mU/cm^2$ to 30 $mU/cm^2$.

**Figure 9** summarizes the results for membranes coated with polydopamine, heparin and, optionally, with chondroitin as a third component in a one-step approach as described in **Example 2**. Minimodules prepared from fibers taken from a commercial Evodial hemodialyzer (Baxter) were tested in parallel with coated MCO Ci-400 membranes coated according to the invention to have a benchmark for heparin activity. The Evodial hemodialyzer contains a heparin-grafted AN69 membrane (HeprAN) composed of a polyacrylonitrile sodium methallylsulfonate copolymer the manufacturing process of which includes a surface treatment with high-molecular-weight polyethyleneimine before heparin grafting. The results obtained in the heparin activity measurements show that, first, heparin can be effectively immobilized

together with polydopamine on the lumen side of the hollow fiber membranes, wherein the resulting heparin activity is comparable to the heparin activity of a commercial heparin-grafted membrane (Evodial), and that, second, sterilizing the minimodules or filters does not have any impact on heparin activity. The presence of a third coating component, in this case chondroitin, only has a slight effect on heparin activity which is potentially due to the competition between chondroitin and heparin for binding to polydopamine.

**Example 4: Influence of the PD-HEP coating on the sieving coefficients of membranes**

Various membranes were used for assessing the effect and influence of a polydopamine-heparin (PD-HEP) coating according to the invention on membranes. Parameters analyzed included, for example, the sieving coefficients for β2-M, myoglobin, complement factor D, YKL-40 and albumin, as well as the Lp of the respective membranes.

**Example 4.1: Comparison of uncoated minimodules with coated minimodules with 6 or 12 mg/mL dopamine and 20 or 40 mg/mL heparin: sieving coefficients**

Hollow fibers as otherwise used in a commercial hemodialyzer (Theranova 400, Baxter Int., Inc.) were used for preparing a minimodule according to **Example 1.1** and were coated in a one-step approach according to **Example 2.1.** As shown in **Table II,** coating with polydopamine and heparin had an influence on the selectivity of the membrane as determined by sieving coefficients for selected marker molecules.

Hollow fibers as otherwise used in experimental filters which have been described before, such as MCO-Ci 400 (see, for example, Boschetti-de-Fierro et al., Scientific Reports (2015) 5: 18448, wherein MCO 4 corresponds to MCO-Ci 400), all from Baxter, were used for preparing minimodules according to **Example 1.1** and were coated in a one-step approach according to **Example 2.1.**

As shown in the following tables, coating with polydopamine and heparin influenced the selectivity performance of the membrane.

*Table II: Influence of differently coating the lumen of hollow fiber membranes from Theranova 400 and MCO Ci-400 with polydopamine and heparin on the sieving coefficients (in %) and the Lp ($\cdot 10^{-4}$cm/bar·s). Coating and testing were done with minimodules. In each case three samples were tested. Average values determined for each set of samples are provided with standard deviation. "SC Alb" refers to the sieving coefficient of albumin, "SC YKL-40" to the sieving coefficient of YKL-40.*

| # | Membrane | Coating | | Average | | |
|---|---|---|---|---|---|---|
| | | Dopamine [mg/mL] | Heparin [mg/mL] | Lp [$\cdot 10^{-4}$ cm/ (bar·s)] | SC Alb | SC YKL-40 |
| 1 | Theranova 400 (Lot 8-6810-H-01) | None | None | 194.33 | 1.53 | 37.21 |
| 2 | Theranova 400 (Lot 9-6601-H-01) | 6 | None | 160.00 | 1.82 | 34.70 |
| 3 | Theranova 400 (Lot 8-6810-H-01) | 6 | 20 | 143.50 | 0.37 | 23.62 |
| | | | | | | |
| 4 | MCO Ci-400 (Lot 4-500-022-11) | None | None | 261.33 | 3.58 | 56.11 |
| 5 | MCO Ci-400 (Lot 4-500-022-11) | 6 | 20 | 203.33 | 0.98 | 40.70 |
| 6 | MCO Ci-400 (Lot 4-500-022-11) | 12 | 20 | 154.33 | 0.53 | 43.79 |
| 7 | MCO Ci-400 (Lot 4-500-022-11) | 12 | 40 | 198.00 | 0.77 | 45.75 |
| 8 | MCO Ci-400 (Lot 4-500-022-11)6 | 6 | 40 | 229.00 | 1.31 | 49.33 |

The results shown in **Table II** show that the coating of the base membrane with both dopamine (polydopamine) and heparin leads to an improved selectivity of the coated membrane compared to the uncoated membrane or to the membrane coated with polydopamine only. For example, the set of data based on MCO Ci-400, which is coated with polydopamine and heparin shows a significant reduction of the sieving coefficient for albumin, whereas the sieving coefficient for YKL-40 is lowered considerably less. In other words, while the membrane lets pass YKL-40, a representative of the group of larger middle molecules, still well compared to the base membrane of the MCO-type, albumin is retained significantly better. Polydopamine only does not have the same effect as the combination of polydopamine and heparin.

The standard coating time used in the experiments performed according to **Example 2** is 1 hour. Increasing the coating (incubation) time seemed to have positive effects in as far as the SC for albumin could be decreased somewhat further, whereas the decrease for the SC of YKL-40 was less pronounced. In terms of industrial applicability shorter coating times will still be preferable. However, also longer coating (incubation) times are feasible, various incubation times can be used depending on the intended outcome.

**Table III** provides for a set of extended data on the influence of a coating according to the invention on the sieving coefficient to β2-M, myoglobin and complement factor D. As can be seen there, the positive effect on sieving coefficients in terms of an increase of selectivity is confirmed for these additional marker molecules.

***Table III:** Influence of coating the lumen of hollow fiber membranes of Theranova 400, MCO Ci-400 with polydopamine and heparin on the sieving coefficients (in %) in comparison with uncoated samples. Coating and testing were done with filters. In each case three samples were tested. "Alb" refers to the sieving coefficient of albumin, "YKL-40" to the sieving coefficient of YKL-40, "MYO" refers to the sieving coefficient of myoglobin, "β2M" refers to the sieving coefficient of β2-M, and "CFD" refers to the sieving coefficient of complement factor D.*

| Membrane | Dopamine (mg/mL) | Heparin (mg/mL) | Average Sieving Coefficient (%) | | | | |
|---|---|---|---|---|---|---|---|
| | | | B2-M | MYO | HSA | YKL-40 | CFD |
| MCO Ci-400 (Lot: 4-500) | None | None | 106.00 | 111,00 | 3.50 | 66.33 | 58.00 |
| MCO Ci-400 (Lot: 4-500) | 12 | 20 | 101.00 | 87.00 | 1.00 | 55.67 | 37.33 |
| MCO Ci-400 (Lot: 4-500) | None | None | 115.60 | 90.37 | 1.57 | 38.57 | 33.63 |
| MCO Ci-400 (Lot: 4-500) | 12 | 20 | 111.17 | 76.20 | 0.53 | 34.03 | 31.57 |
| Theranova 400 (Lot: 9-6605-H-01) | None | None | 95.00 | 80.33 | 0.90 | 35.00 | 50.33 |
| Theranova 400 (Lot: 9-6605-H-01) | 6 | 20 | 97.33 | 68.00 | 0.63 | 37.33 | 50.67 |

In another experiment, mini-modules prepared from Revaclear high-flux (HF) membranes containing either 177 or 355 fibers were successively coated, in a two-step approach, with polydopamine and heparin. The polydopamine coating solution was prepared shortly before starting the coating procedure to synchronize the polymerization of the dopamine and the coating of the membrane surface. The polydopamine coating solution contained 0.5 g/L dopamine in 10 mM tris buffer adjusted to a pH of 8.5 with HCl. For heparin, a buffer solution of 50 mM acetic acid and 50 mM sodium acetate in deionized (DI) water at pH 4.5 and 0.5 mM NaCl was prepared. Heparin was added in a concentration of 1 mg/mL. The shell side of the module surrounding the outer surface of the membranes is filled with tris buffer, which is adjusted to pH 8.5 with HCl. The polydopamine coating solution was pumped through the lumen of the fibers. All openings of the module were then closed, and the polymerization of polydopamine was carried out for 24 h in the filled membrane module. Finally, the lumen and the shell side were flushed three times with DI water. In a subsequent step, heparin is immobilized on the polydopamine-coated hollow fiber membranes. On the lumen side, the hollow fiber membrane module is filled with the heparin coating solution containing 1 mg/mL heparin and the shell side is filled with sodium acetate buffer. After 24 h, the hollow fiber membranes are flushed with DI water on the lumen and shell side. For comparing uncoated (HF), partially (with polydopamine (HF-PD) and fully coated (with polydopamine and heparin, HF-PD-HEP) modules were prepared and tested.

**Figure 5** summarizes the test results of the Lp (provided as "pure water permeance (PWP)" and the sieving coefficients of myoglobin as well as albumin at a physiological temperature of 37°C for the membrane modules HF, HF-PD, and HF-PD-HEP. The Lp measurement in this case was run with DI water with a transmembrane pressure (TMP) of 1 bar. The membrane modules were measured in a dead-end filtration. The measurement was started after continuous permeate flux (J) was reached. The permeating water corresponds to the PWP of the membrane in L/($m^2$ h bar), short LMH/bar. The reference membrane HF-PM-D0L has a PWP of around 318 300 LMH/bar. The sieving coefficients of myoglobin and albumin were 87 % and 2.5 %. In comparison, the polydopamine coated fiber HF-PD features a higher Lp of around 350 LMH/bar. The additional coating with polydopamine hydrophilizes the already hydrophilic PES membrane surface and thus results in a higher PWP. The polydopamine layer also has an influence on the sieving coefficients. The sieving coefficient of myoglobin increases with the polydopamine coating to 93 %, while the sieving coefficient of albumin remains almost constant with 3.5 %. The HF-PD-HEP shows an even higher PWP of around 420 LMH/bar. However, its sieving coefficients for myoglobin reaches 100 % with an albumin sieving coefficient of still 3.5 %. Compared to HF-PD HF-PD-HEP has a higher PWP of 418 LMH/bar. The increased hydrophilicity due to the presence of heparin probably leads to the PWP difference. The effect of higher PWP of polydopamine immobilized membranes modified with heparin was already reported by Ma et al. The myoglobin sieving coefficient with 100 %, however, is 7 % higher compared to HF-PD, while the albumin sieving coefficient remains the same. The immobilized polydopamine and heparin thus seem to lead to a different surface charge and the chemical composition of the membrane surface. Without wanting to be bound by theory, the surface modification seems to result in a changed driving force, as well as a higher PWP,

which may be reasons for higher sieving coefficients of myoglobin. The results show that the coating functionalizes the membrane surface successfully and at the same time improves the sieving characteristics for dialysis applications, revealing a high retention for albumin and better removal of myoglobin at the same time. While only changing the membrane transport properties slightly, the proposed modification procedure is a novel way to prepare heparin coatings directly on, for example, PES membranes, which improves the dialysis treatment.

**Example 5: Clearance rates achieved with uncoated membranes and membranes coated with PD-HEP**

Average clearance rates for uncoated and coated samples (filters) were determined as described in **Example 1.6** in order to better understand the influence of a modification or coating according to the invention on the clearance performance of filters. Various membrane types as described in **Example 4.1** were coated as described in Example 2 and clearance rates were determined with the resulting filters.

**Example 6: Heparin activity after e-beam sterilization**

As described in **Example 2,** membranes can be coated with a combination of polydopamine and heparin. In addition to increasing selectivity, such coatings have also the ability to reduce the thrombogenicity of the resulting membranes and filters. It is therefore important to maintain the activity of heparin during sterilization of a coated membrane and device.

Accordingly, heparin activity was tested after e-beam sterilization as described above. Heparin activity can be determined, for example, with the heparin anti-Xa assay. The heparin anti-Xa assay is based on the ability of heparin to inhibit the activity of activated factor X (Xa) in the reagent. The reagent includes excess antithrombin, making the heparin in the sample the rate-limiting reagent for Xa inhibition. Heparin in the sample inhibits the enzymatic conversion of a Xa-specific chromogenic substrate to colored product by factor Xa. Standard curves are used to calculate heparin activity in the sample in $mU/cm^2$. The test has been widely used and can be performed according to Newall F., Anti-factor Xa (anti-Xa) assay. Methods Mol Biol. 2013;992:265-72.

It was found that heparin remains stable and active after e-beam sterilization, see **Figure 9.**

**Example 7: C3a Activation and hemocompatibility of coated devices according to the invention**

Activation of the complement system by, for example, a medical device that contacts blood, can have deleterious effects including tissue damage and inflammation. The complement system is comprised of many small proteins and many of them can be used as markers for complement activation. Measurement of, for example, C3a, is suggested by ISO 10993-4.

Minimodules are coated with 12 mg/mL dopamine and 40 mg/mL heparin as described in **Example 2.1.** The same minimodules are left uncoated to have a reference. Minimodules comprising Cuprophan (regenerated cellulose) hollow fibers are prepared and used as a positive control. Cuprophan membranes are symmetrically structured and are extremely polar and hydrophilic due to their high proportion of hydroxyl groups. It is known that regenerated cellulose membranes are less biocompatible than modern synthetic membranes such as the MCO Ci-400 membranes. C3a activation is determined with the MicroVue C3a Plus enzyme immunoassay (Quidel, U.S.A.) for determining human C3a concentration in plasma and according to the protocol. The test principle is the quantitative in-vitro determination of the C3a concentration in human plasma, wherein test samples are added to a microtiter plate which is coated with murine monoclonal antibodies which are specific for human C3a. C3a which is available in the test sample is immobilized during incubation. After a washing step, a chromogenic substrate solution (TMB) is added. The bound peroxidase reacts with the substrate and forms a blue color. After stopping the reaction chemically, a blue color appears. The color intensity is proportional to the C3a concentration and can be determined photometrically at 450 nm.

It was found that base membranes modified and/or coated with polydopamine and heparin decrease C3 activation and are at least as or more hemocompatible than membranes of the state of the art. Accordingly, the coating as described herein provides for an excellent hemocompatibility and recommends using a coating according to the invention with hemodialysis membranes and filtration devices for blood treatment.

**Example 8: Impact of sterilization on coated filters or minimodules**

As mentioned before, coated filters or minimodules can be sterilized with various methods, such as steam, e-beam or gamma ray sterilization. Preferably, e-beam or gamma ray sterilization is used as it was found that these sterilization methods do not negatively impact the improved filter performance and the integrity of the PD-HEP coating, whereas steam sterilization tends to negatively impact the increase in selectivity that is achieved with the coating according to the invention.

**Example 9: Coating in recirculation mode**

Coating of minimodules in recirculation mode as described in **Example 2** and **Figure 4** resulted in equivalent coatings and effects on membrane selectivity in comparison with, for example, an incubation approach wherein e.g., the lumen of hollow fibers is filled with a coating solution and incubated for a given time before removing the solution and rinsing the fibers. Coating is, accordingly, largely independent of the method used and can be adapted to the requirements of the production process for filters.

## Claims

1. A method for increasing the selectivity of a membrane for use in blood treatment comprising the steps of

   (i) providing a base membrane and
   (ii) coating the base membrane with polydopamine and heparin.

2. A method according to claim 1, wherein the base membrane comprises a blend of i) a polysulfone (PS), polyethersulfone (PES) or polyarylethersulfone (PAES) and ii) polyvinylpyrrolidone (PVP).

3. A method according to claim 1 or claim 2, wherein the base membrane of step (i) further comprises polydopamine and is coated in step (ii) with heparin.

4. A method according to claim 1, wherein the base membrane is selected from sulfonated polyacrylonitrile (PAN) membranes, poly(methyl methacrylate) (PMMA) membranes, cellulose triacetate (CTA) membranes, or ethylene vinyl alcohol copolymer (EVAL) membranes.

5. A method according to any of claims 1, 2 or 4, wherein the base membrane is first coated with polydopamine, followed by coating with heparin.

6. A method according to any of claims 1, 2 or 4, wherein the base membrane is coated with a mixture comprising polydopamine and heparin.

7. A method according to any of claims 1, 2 and 4 to 6, wherein the base membrane is coated with polydopamine in an amount of from 0.30 $\mu g/cm^2$ to 1.10 $\mu g/cm^2$ of the membrane surface.

8. A method according to any of claims 1 to 7, wherein the base membrane is coated with heparin in an amount of from 5 $mU/cm^2$ to 100 $mU/cm^2$ of the membrane surface.

9. A method according to any of claims 1 to 8, wherein the base membrane has a MWRO of from 8.5 to 14.0 kDa and a MWCO of from 50.0 to 130.0 kDa as measured by dextran sieving before blood contact.

10. A method according to any of claims 1 to 9, wherein the base membrane has a MWRO of from 15kDa to 20kDa and a MWCO of from 170kDa to 320kDa as measured by dextran sieving before blood contact.

11. A method according to any of claims 1 to 10, wherein the membrane has a MWRO of from 5.0 kDa to 8.5 kDa and a MWCO of from 25 kDa to 50 kDa as measured by dextran sieving before blood contact.

12. A method according to any of claims 1 to 11, wherein the membrane is a flat sheet membrane or a hollow fiber membrane.

13. A method according to claim 1, wherein step (ii) comprises the steps of

   (a) Providing a solution comprising dopamine in an amount of from 2 mg/mL to 80 mg/mL at a pH of from 8.0 to 10.0;
   (b) Adding heparin to the solution of step (a) in an amount of from 0.1 mg/mL to 100 mg/mL;
   (c) Contacting the base membrane surface with the solution of step (b);
   (d) Rinsing and optionally drying the coated base membrane of step (c);
   (e) Optionally sterilizing the coated base membrane of step (c).

**14.** A method according to claim 1, wherein step (ii) comprises the steps of

(a) Providing a solution comprising dopamine in an amount of from 2 mg/mL to 80 mg/mL and heparin in an amount of from 5 mg/mL and 80 mg/mL at a pH of from 8.0 to 10.0;
(b) Contacting the base membrane surface with the solution of step (a);
(c) Rinsing and optionally drying the coated base membrane of step (b);
(d) Optionally sterilizing the coated base membrane of step (c).

**15.** A method according to claim 3, wherein the base membrane of step (i) is provided by

(a) Forming a polymer solution comprising (i) at least one of polysulfone, polyethersulfone or polyarylethersulfone, ii) polyvinylpyrrolidone, and (iii) dopamine;
(b) Extruding the polymer solution through an outer ring slit of a nozzle with two concentric openings into a precipitation bath; and simultaneously
(c) Extruding a center fluid through the inner opening of the nozzle;
(d) Washing the obtained membrane, optionally followed by drying;

wherein the polymer solution comprises from 10 to 15 wt.%, relative to the total weight of the polymer solution, of polysulfone, polyethersulfone or polyarylethersulfone; from 1 to 10 wt.%, relative to the total weight of the polymer solution, of polyvinylpyrrolidone; and from 0.3 to 0.8 wt.%, relative to the total weight of the solution, of dopamine.

**16.** A method according to claim 15, wherein the center fluid comprises $H_2O$ and tris buffer at a concentration of from 5 mM to 20 mM and has a pH of from 8.0 and 9.5.

**17.** A method according to claim 15 or claim 16, wherein following step (d), the polydopamine containing membrane is

(e) Contacted with a solution comprising heparin in an amount of from 5 mg/mL and 80 mg/mL, followed by
(f) Rinsing and optionally drying the heparin coated base membrane of step (e); and
(g) Optionally sterilizing the heparin coated base membrane of step (f).

**18.** A dialyzer for blood treatment comprising a membrane that has been treated by a method according to any of claims 1 to 17.

**19.** A membrane comprising a blend of (i) at least one of polysulfone, polyethersulfone or polyarylethersulfone, ii) polyvinylpyrrolidone, and (iii) dopamine and which is coated with heparin in amount of from 5 $mU/cm^2$ to 100 $mU/cm^2$ of the membrane surface.

**20.** A dialyzer for blood treatment comprising the membrane of claim 19.

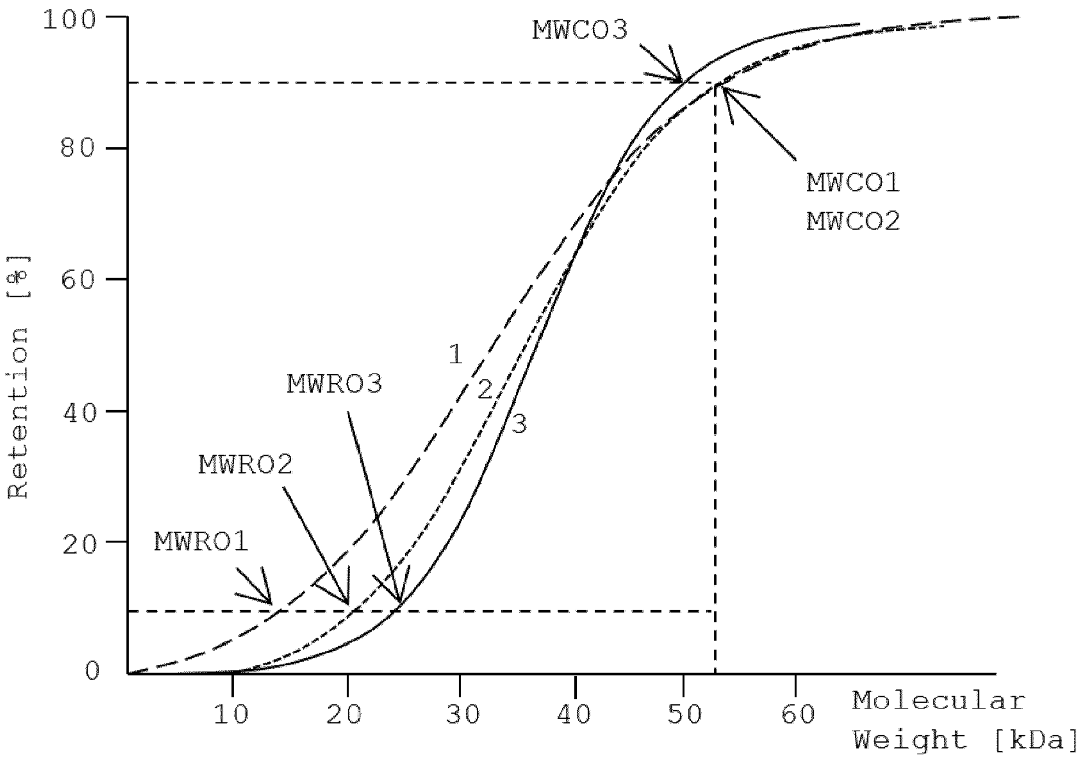
100
80
60
40
20
0
Retention [%]
MWCO3
MWCO1
MWCO2
1
2
3
MWRO3
MWRO2
MWRO1
10
20
30
40
50
60
Molecular Weight [kDa]

**Figure 1**

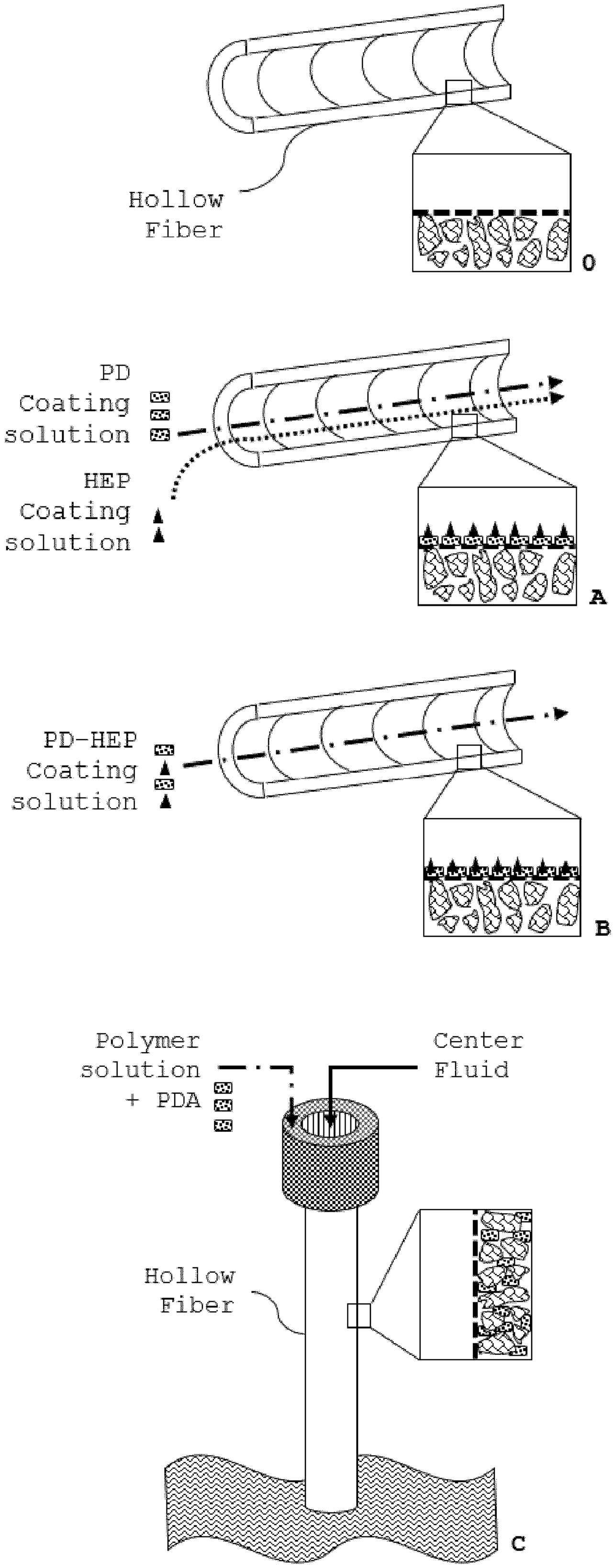
Hollow
Fiber
0
PD
Coating
solution
HEP
Coating
solution
A
PD-HEP
Coating
solution
B
Polymer
solution
+ PDA
Center
Fluid
Hollow
Fiber
C

Figure 2

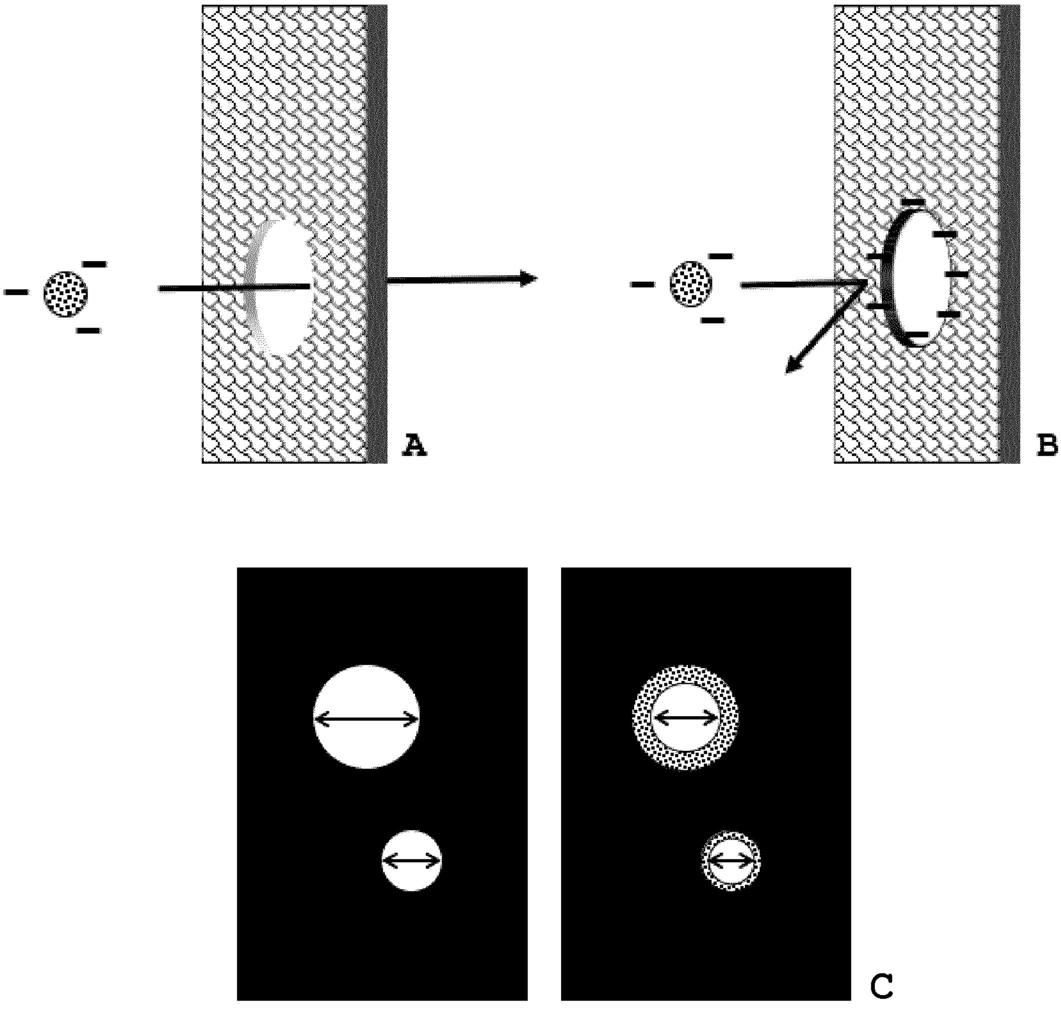


Figure 3

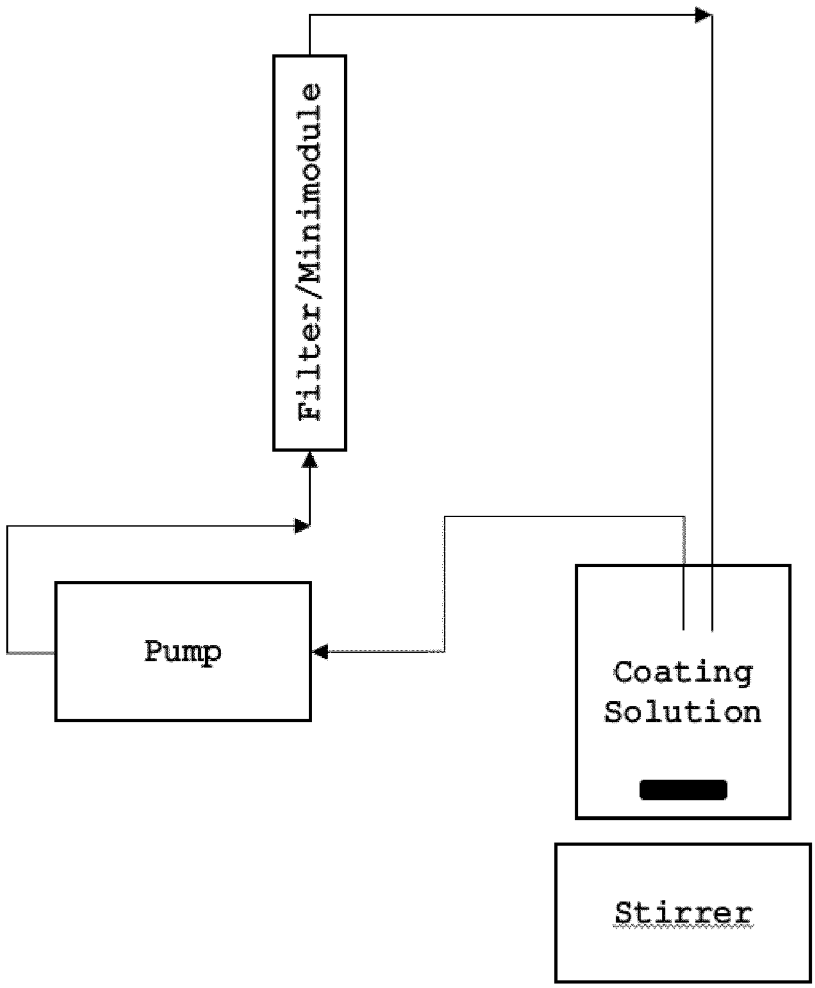


Figure 4

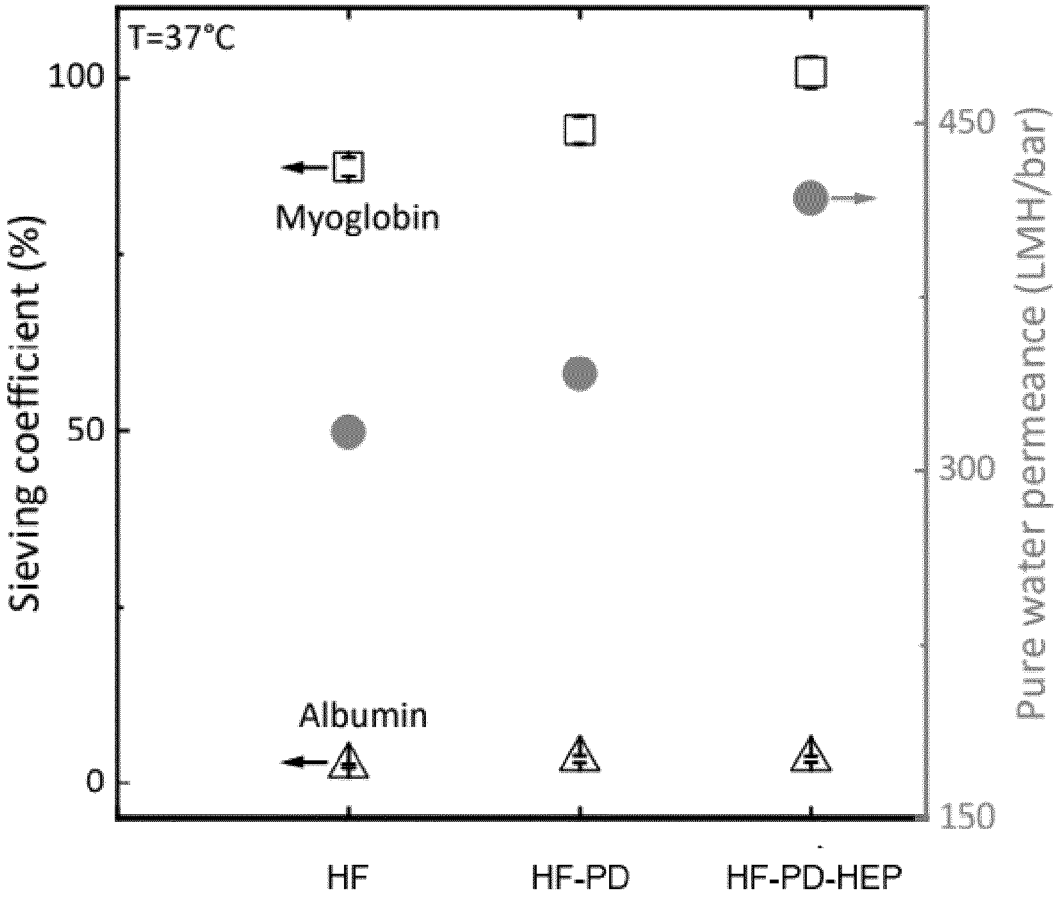
T=37°C
100
50
0
Sieving coefficient (%)
Myoglobin
Albumin
450
300
150
Pure water permeance (LMH/bar)
HF
HF-PD
HF-PD-HEP

Figure 5

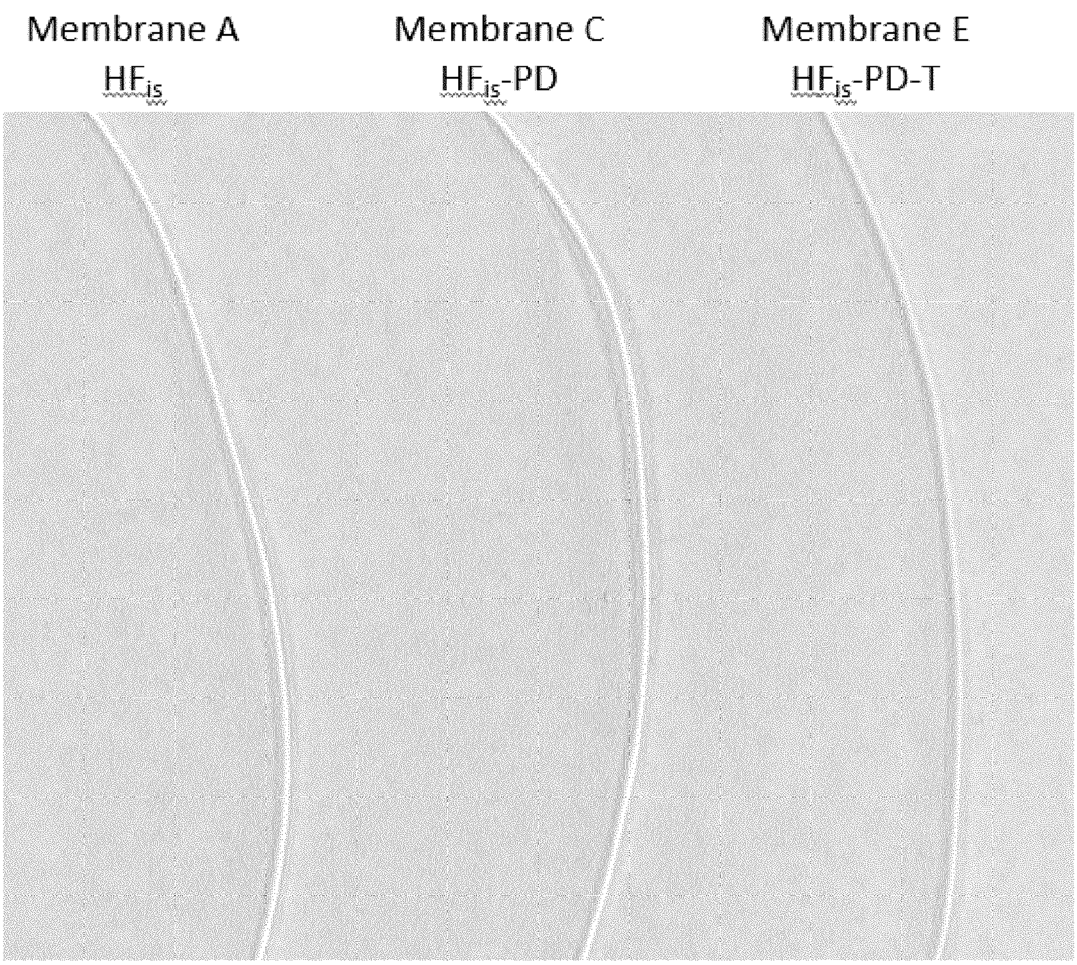


Figure 6

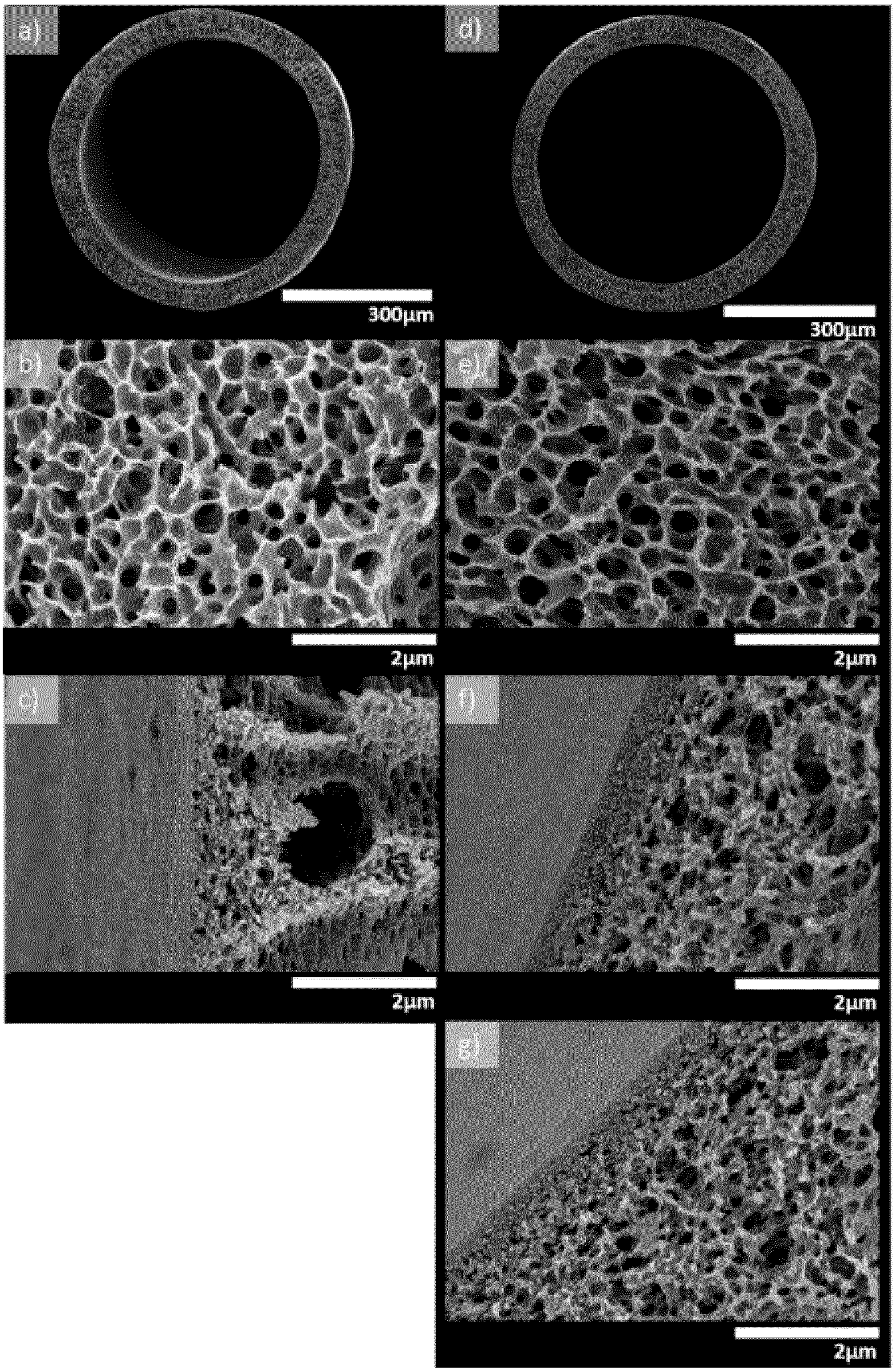


Figure 7

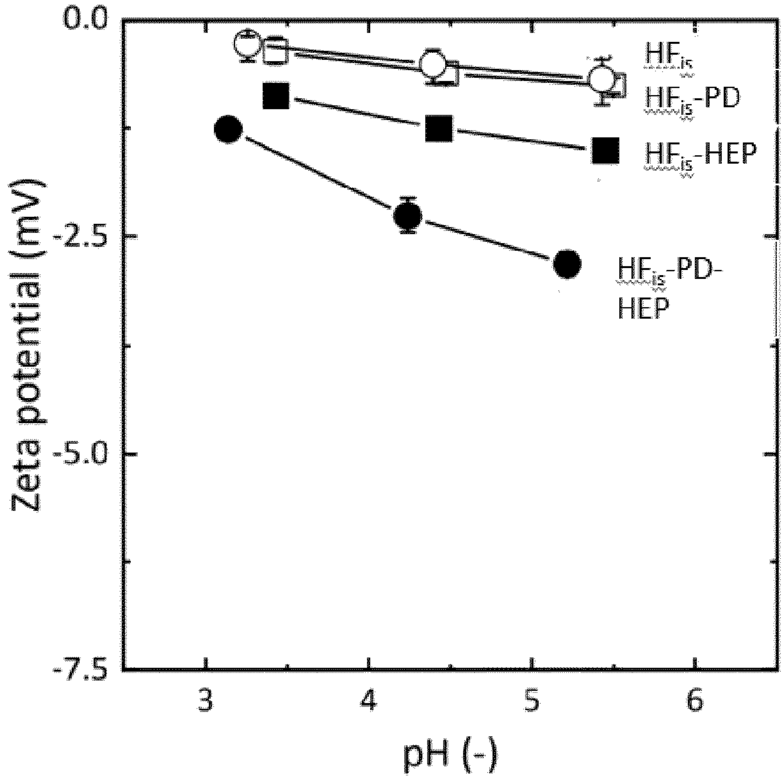
0.0
-2.5
-5.0
-7.5
Zeta potential (mV)
3
4
5
6
pH (-)
HFis
HFis-PD
HFis-HEP
HFis-PD-HEP

A

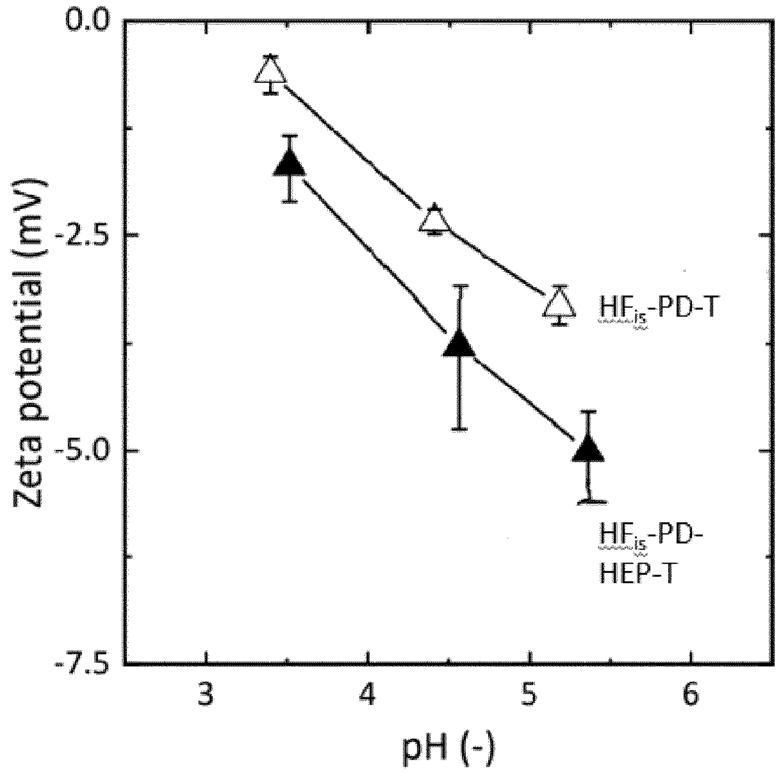
0.0
-2.5
-5.0
-7.5
Zeta potential (mV)
3
4
5
6
pH (-)
HFis-PD-T
HFis-PD-HEP-T

B

Figure 8

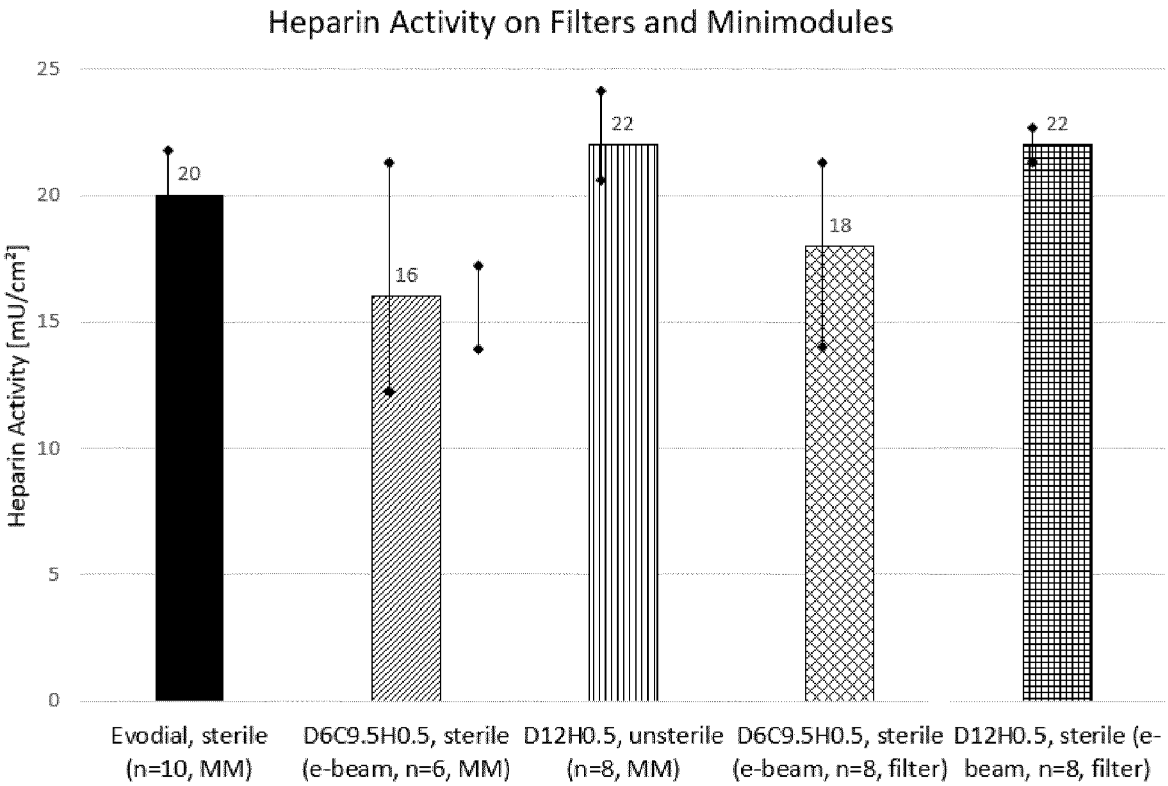
Heparin Activity on Filters and Minimodules
Heparin Activity [mU/cm²]
25
20
15
10
5
0
20
16
22
18
22
Evodial, sterile (n=10, MM)
D6C9.5H0.5, sterile (e-beam, n=6, MM)
D12H0.5, unsterile (n=8, MM)
D6C9.5H0.5, sterile (e-beam, n=8, filter)
D12H0.5, sterile (e-beam, n=8, filter)

Figure 9

Europäisches Patentamt
European Patent Office
Office européen des brevets

# EUROPEAN SEARCH REPORT

Application Number
EP 21 21 6677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIE BINGWU ET AL: "A Decoration of heparin and bovine serum albumin on polysulfone membrane assisted via polydopamine strategy for hemodialysis", JOURNAL OF BIOMATERIALS SCIENCE. POLYMER EDITION, VSP, UTRECHT, NL , vol. 27, no. 9 12 June 2016 (2016-06-12), pages 880-897, XP009535996, ISSN: 0920-5063, DOI: 10.1080/09205063.2016.1169479 Retrieved from the Internet: URL:http://www.tandfonline.com/doi/full/10.1080/09205063.2016.1169479 [retrieved on 2016-04-22] | 1,2,4,5, 7-14,18 | INV. B01D67/00 A61M1/34 B01D71/44 B01D71/68 |
| Y | * abstract * * paragraphs [02.1] - [02.2] * ----- | 3,15-17, 19,20 | |
| X | GAO AILIN ET AL: "Preparation and evaluation of heparin-immobilized poly (lactic acid) (PLA) membrane for hemodialysis", JOURNAL OF MEMBRANE SCIENCE, vol. 452, 1 February 2014 (2014-02-01), pages 390-399, XP55926240, NL ISSN: 0376-7388, DOI: 10.1016/j.memsci.2013.10.016 | 1,2,4,5, 7-14,18 | TECHNICAL FIELDS SEARCHED (IPC) B01D A61M |
| Y | * abstract * * paragraphs [02.2] - [02.3] * ----- -/-- | 3,15-17, 19,20 | |

The present search report has been drawn up for all claims

1

| Place of search | Date of completion of the search | Examiner |
|---|---|---|
| Munich | 1 June 2022 | Veríssimo, Sónia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 21 6677

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MA ET AL.: "Mussel-inspired self-coating at macro-interface with improved biocompatibility and bioactivity via dopamine grafted heparin-like polymers and heparin", JOURNAL OF MATERIALS CHEMISTRY B, vol. 2, 2014, pages 363-375, XP002806663, * abstract * * paragraphs [02.2] - [02.3] * ----- | 1,2,4, 6-10, 12-14,18 | |
| X | JIANG J H ET AL: "Surface modification of PE porous membranes based on the strong adhesion of polydopamine and covalent immobilization of heparin", JOURNAL OF MEMBRANE SCIENCE, ELSEVIER BV, NL, vol. 364, no. 1-2, 15 November 2010 (2010-11-15), pages 194-202, XP027450293, ISSN: 0376-7388, DOI: 10.1016/J.MEMSCI.2010.08.017 [retrieved on 2010-08-14] | 1,2,4,5, 7-14,18 | TECHNICAL FIELDS SEARCHED (IPC) |
| Y | * abstract * * paragraphs [02.1] - [02.2] * ----- | 3,15-17, 19,20 | |
| Y | CN 103 480 278 B (YANTAI LVSHUIFU MEMBRANE MATERIAL LTD) 25 February 2015 (2015-02-25) * claims * ----- | 3,15-17, 19,20 | |
| A | WO 2015/118046 A1 (GAMBRO LUNDIA AB [SE]) 13 August 2015 (2015-08-13) * claims * ----- | 1-20 | |

The present search report has been drawn up for all claims

1

| Place of search | Date of completion of the search | Examiner |
|---|---|---|
| Munich | 1 June 2022 | Veríssimo, Sónia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO.

EP 21 21 6677

This annex lists the patent family members relating to the patent documents cited in the above-mentioned European search report. The members are as contained in the European Patent Office EDP file on
The European Patent Office is in no way liable for these particulars which are merely given for the purpose of information.

01-06-2022

| Patent document cited in search report | Publication date | Patent family member(s) | Publication date |
|---|---|---|---|
| CN 103480278 B | 25-02-2015 | NONE | |
| WO 2015118046 A1 | 13-08-2015 | AU 2015214950 A1 | 04-08-2016 |
| | | AU 2018278913 A1 | 03-01-2019 |
| | | CA 2938222 A1 | 13-08-2015 |
| | | CN 105722582 A | 29-06-2016 |
| | | CN 111545068 A | 18-08-2020 |
| | | EP 3102312 A1 | 14-12-2016 |
| | | EP 3427814 A1 | 16-01-2019 |
| | | ES 2701847 T3 | 26-02-2019 |
| | | HK 1224246 A1 | 18-08-2017 |
| | | JP 6636437 B2 | 29-01-2020 |
| | | JP 6924253 B2 | 25-08-2021 |
| | | JP 2017507706 A | 23-03-2017 |
| | | JP 2020039952 A | 19-03-2020 |
| | | KR 20160119190 A | 12-10-2016 |
| | | KR 20210129258 A | 27-10-2021 |
| | | PL 3102312 T3 | 31-05-2019 |
| | | PT 3102312 T | 18-12-2018 |
| | | US 2017165615 A1 | 15-06-2017 |
| | | US 2020316533 A1 | 08-10-2020 |
| | | US 2022152563 A1 | 19-05-2022 |
| | | WO 2015118046 A1 | 13-08-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015118046 A1
- US 20030021826 A1
- WO 2012047282 A2
- US 20100059433 A1
- WO 2015118045 A1
- WO 2004056460 A1


### Non-patent literature cited in the description


- **BOSCHETTI-DE-FIERRO et al.** *Scientific Reports,* 2015, vol. 5, 18448
- **ROSNER et al.** Classification of Uremic Toxins and Their Role in Kidney Failure. *CJASN,* 2021, vol. 16, 1-8
- **LORENZ et al.** *Kidney International,* 2018, vol. 93, 221-230
- **KIRSCH et al.** *Nephrology Dialysis Transplantation,* 2017, vol. 32, 165
- **LIEBSCHER.** Chemistry of Polydopamine - Scope, Variation, and Limitation. *Eur. J. Org. Chem.,* 2019, vol. 2019, 4976-4994
- **LI et al.** *Desalination,* 2014, vol. 344, 422-430
- **ZHU et al.** Surface modification of PVDF porous membranes via poly(DOPA) coating and heparin immobilization, Colloids and Surfaces B. *Biointerfaces,* 2009, vol. 69 (1), 152-155
- **WHILE JIANG et al.** Surface modification of PE porous membranes based on the strong adhesion of polydopamine and covalent immobilization of heparin. *Journal of Membrane Science,* 2010, vol. 364 (1-2), 194-202
- **YOU et al.** Enhancement of blood compatibility of poly (urethane) substrates by mussel-inspired adhesive heparin coating. *Bioconjugate Chemistry,* 2011, vol. 22 (7), 1264-1269
- **GAO et al.** *Journal of Membrane Science,* 2014, vol. 452, 390-399
- **MA et al.** Mussel-inspired self-coating at macro-interface with improved biocompatibility and bioactivity via dopamine grafted heparin-like polymers and heparin. *Journal of Materials Chemistry B,* 2014, vol. 2, 363-375
- **RONCO ; CLARK.** *Nature Reviews. Nephrology,* 2018, vol. 14 (6), 394-410
- **GONDOUIN ; HUTCHISON.** High Cut-Off Dialysis Membranes: Current Uses and Future Potential. *Advances in Chronic Kidney Disease,* 2011, vol. 18, 180-187
- **ROSNER et al.** Classification of Uremic Toxins and Their Role in Kidney Failure. *Clin J Am Soc Nephrol.,* 2021
- **SALOMÄKI et al.** *J. Phys. Chem. B,* 2018, vol. 122, 6314-6327
- **LANER-PLAMBERGER et al.** *Int. J. Mol. Sci.,* 2021, vol. 22, 12041
- **SAID et al.** A Review of Commercial Developments and Recent Laboratory Research of dialyzers and Membranes for Hemodialysis Application. *Membranes,* 2021, vol. 11, 767
- **RONCO ; CLARK.** Nature Reviews. *Nephrology,* 2018, vol. 14 (6), 394-410
- **SMITH et al.** *Analytical Biochemistry,* 1985, vol. 150, 76-85
- **LAMMERS et al.** A comparison of seven methods to analyze heparin in biomaterials: quantification, location, and anticoagulant activity. *Tissue Eng Part C Methods.,* 2011, vol. 17 (6), 669-76
- **NEWALL F.** Anti-factor Xa (anti-Xa) assay. *Methods Mol Biol.,* 2013, vol. 992, 265-72